(19) Europäisches Patentamt
European Patent Office
Office européen des brevets

(11) EP 2 193 147 B1

# (12) EUROPEAN PATENT SPECIFICATION

(45) Date of publication and mention of the grant of the patent:
**08.04.2015 Bulletin 2015/15**

(21) Application number: **07858965.2**

(22) Date of filing: **19.09.2007**

(51) Int Cl.:
***C07K 16/18*** *(2006.01)* ***A61K 39/395*** *(2006.01)*
***A61K 47/48*** *(2006.01)* ***A61P 35/00*** *(2006.01)*

(86) International application number:
**PCT/IB2007/003786**

(87) International publication number:
**WO 2009/037525 (26.03.2009 Gazette 2009/13)**

## (54) NUCLEOTIDE AND PROTEIN SEQUENCES OF AN ANTIBODY DIRECTED AGAINST AN EPITOPE COMMON TO HUMAN ACIDIC AND BASIC FERRITINS, MONOCLONAL ANTIBODIES OR ANTIBODY-LIKE MOLECULES COMPRISING THESE SEQUENCES AND USE THEREOF

NUKLEOTID- UND PROTEINSEQUENZEN EINES GEGEN EIN HUMANEN SAUREN UND BASISCHEN FERRITINEN EIGENEN EPITOP GERICHTETEN ANTIKÖRPERS, MONOKLONALE ANTIKÖRPER ODER ANTIKÖRPERÄHNLICHE MOLEKÜLE, DIE DIESE SEQUENZEN ENTHALTEN, UND DEREN VERWENDUNG

SÉQUENCES NUCLÉOTIDIQUES ET PROTÉIQUES D'UN ANTICORPS DIRIGÉ CONTRE UN ÉPITOPE COMMUN AUX FERRITINES ACIDES ET BASIQUES HUMAINES, ANTICORPS MONOCLONAUX OU MOLÉCULES DE TYPE ANTICORPS COMPRENANT CES SÉQUENCES ET LEUR UTILISATION

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**09.06.2010 Bulletin 2010/23**

(73) Proprietor: **Immune Pharmaceuticals Ltd.**
**46733 Herzliya-Pituach (IL)**

(72) Inventors:
- **KADOUCHE, Jean**
  **F-75013 Paris (FR)**
- **SABBAH-PETROVER, Emmanuelle**
  **F-75017 PARIS (FR)**
- **CHOSE, Olivier**
  **F-92150 SURESNES (FR)**

(74) Representative: **Desaix, Anne et al**
**Ernest Gutmann - Yves Plasseraud S.A.S.**
**3, rue Auber**
**75009 Paris (FR)**

(56) References cited:
**EP-A- 1 563 851** **WO-A-01/52889**
**WO-A-2007/034479**

- **SABBAH ET AL: "In vitro and in vivo comparison of DTPA- and DOTA-conjugated antiferritin monoclonal antibody for imaging and therapy of pancreatic cancer" NUCLEAR MEDICINE AND BIOLOGY, ELSEVIER, NY, US, vol. 34, no. 3, 23 March 2007 (2007-03-23), pages 293-304, XP005935426 ISSN: 0969-8051**
- **GOLDSTEIN ET AL: "The design and evaluation of a novel targeted drug delivery system using cationic emulsion-antibody conjugates" JOURNAL OF CONTROLLED RELEASE, ELSEVIER, AMSTERDAM, NL, vol. 108, no. 2-3, 28 November 2005 (2005-11-28), pages 418-432, XP005163083 ISSN: 0168-3659**
- **VRIESENDORP H M ET AL: "Recurrence of Hodgkin's disease after indium-111 and yttrium-90 labeled antiferritin administration." CANCER 15 DEC 1997, vol. 80, no. 12 Suppl, 15 December 1997 (1997-12-15), pages 2721-2727, XP002484635 ISSN: 0008-543X**
- **ONO K ET AL: "COMMON EPITOPES IN HUMAN ISOFERRITINS CHARACTERIZED BY MURINE MONOCLONAL ANTIBODIES", JOURNAL OF BIOCHEMISTRY, JAPANESE BIOCHEMICAL SOCIETY / OUP, TOKYO; JP, vol. 99, no. 1, 1 January 1986 (1986-01-01), pages 269-279, XP000930082, ISSN: 0021-924X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **KETTLEBOROUGH C A ET AL: "HUMANIZATION OF A MOUSE MONOCLONAL ANTIBODY BY CDR-GRAFTING: THE IMPORTANCE OF FRAMEWORK RESIDUES ON LOOP CONFORMATION", PROTEIN ENGINEERING, OXFORD UNIVERSITY PRESS, SURREY, GB, vol. 4, no. 7, 1 October 1991 (1991-10-01) , pages 773-783, XP002003434, ISSN: 0269-2139**
- **TABRIZI M ET AL: "Biodistribution mechanisms of therapeutic monoclonal antibodies in health and disease", AAPS JOURNAL 2010 SPRINGER NEW YORK USA, vol. 12, no. 1, March 2010 (2010-03), pages 33-43, ISSN: 1550-7416**
- **LEVÊQUE DOMINIQUE ET AL: "Pharmacokinetics of therapeutic monoclonal antibodies used in oncology.", ANTICANCER RESEARCH 2005 MAY-JUN, vol. 25, no. 3c, May 2005 (2005-05), pages 2327-2343, ISSN: 0250-7005**
- **SAIFUL ALAM A F: "Radioimmunotherapy of human hepatocellular carcinoma xenografts with 131I-labelled antiferritin antibody.", BRITISH JOURNAL OF CANCER APR 1991, vol. 63, no. 4, April 1991 (1991-04), pages 503-507, ISSN: 0007-0920**
- **PATRICK CHAMES ET AL: "Therapeutic antibodies: successes, limitations and hopes for the future", BRITISH JOURNAL OF PHARMACOLOGY, NATURE PUBLISHING GROUP, BASINGSTOKE, HANTS; GB; BIOSCIENCES INFORMATION SERVICE, PHILADELPHIA, PA, US, vol. 157, no. 2, 1 May 2009 (2009-05-01), pages 220-233, XP002714017, ISSN: 0007-1188, DOI: 10.1111/J. 1476-5381.2009.00190.X**
- **ANNEX I - SUMMARY OF PRODUCT CHARACTERISTICS (Erbitux 5 mg/mL solution for infusion)**
- **ANNEX I - SUMMARY OF PRODUCT CHARACTERISTICS (Herceptin 150 mg powder for concentrate solution for infusion)**
- **SALFELD JOCHEN G: "Isotype selection in antibody engineering", NATURE BIOTECHNOLOGY, NATURE PUBLISHING GROUP, UNITED STATES, vol. 25, no. 12, 1 December 2007 (2007-12-01), pages 1369-1372, XP002668461, ISSN: 1546-1696**
- **LAI J ET AL: "Pharmacokinetics of radiolabeled polyclonal antiferritin in patients with Hodgkin's disease.", CLINICAL CANCER RESEARCH : AN OFFICIAL JOURNAL OF THE AMERICAN ASSOCIATION FOR CANCER RESEARCH OCT 1999, vol. 5, no. 10 Suppl, October 1999 (1999-10), pages 3315s-3323s, ISSN: 1078-0432**
- **IGAWA TOMOYUKI ET AL: "Engineering the variable region of therapeutic IgG antibodies", MABS, LANDES BIOSCIENCE, US, vol. 3, no. 3, 1 May 2011 (2011-05-01), pages 243-252, XP009153597, ISSN: 1942-0870**


Remarks:

The file contains technical information submitted after the application was filed and not included in this specification

## Description

### Field of the Invention

The present application relates to chimeric and humanized monoclonal antibodies, fragments thereof and antibody-like molecules that recognize an epitope common to human acidic and basic isoferritins. These chimeric and humanized anti-ferritin monoclonal antibodies, fragments thereof and antibody-like molecules can be used in pharmaceutical compositions for therapy to target various cancer cells in a mammal. A method for delivering anti-ferritin monoclonal antibodies, fragments thereof and antibody-like molecules to cancerous lymph cells, pancreatic cells, lymphatic endothelium cells, and liver cells is also disclosed, as well as methods for treating pancreatic cancer, hepatocellular carcinomas, Kaposi's sarcoma and Hodgkin's lymphoma.

### BACKGROUND OF THE INVENTION

It is well known that the immune system plays a role in cancer progression. Immunotherapy is a fairly new but promising weapon in the arsenal of anticancer treatments. Cancer immunotherapy aims at enhancing the body's natural ability to defend itself against malignant tumors through stimulation of the patient immune system via various pharmacological agents such as vaccines, T cell infusions, cytokine infusions or antibodies. These pharmaceutical agents can act by stimulating an antitumor response by increasing the number of effector cells, by producing lymphokines, by decreasing suppressor mechanisms, by improving tolerance to cytotoxic drugs or chemotherapy and/or by altering tumor cells to increase their immunogenicity.

Monoclonal antibodies can act either indirectly by recruiting cells, which is know as antibody-dependent cell mediated cytotoxicity (ADCC), or by triggering directly cell death, which is known as complement dependent toxicity (CDC).

An example of antibody-dependent cell mediated toxicity (ADCC) is that induced by rituximab, a chimeric antibody that targets the CD20 antigen, expressed on a significant number of B cell malignancies. The constant part (Fc) of the antibody binds to the Fc receptors found on monocytes, macropages and natural killer cells, which leads to destruction of the tumor cells. The monocytes, macrophages and natural killer cells can in turn engulf the bound tumor cells and destroy it. Natural killer cells also secrete cytokines that then leads to cell death.

In complement dependent cytotoxicity (CDC), the monoclonal antibody binds to its receptor on the tumor cells and initiates the complement system, which causes cell membrane permeation leading inevitably to cell death.

In yet another scenario, monoclonal antibodies can kill cells by blocking growth mechanisms or by triggering apoptosis.

Hence, antibodies can act by different ways to treat cancer. Moreover, they can either be used alone as described above or they can be coupled to toxins, cytotoxic agents, drugs, immune killer cells or radioisotopes to deliver a cytotoxic substance or compound at the tumor sites. Examples of radioisotopes that can be used in radioimmunotherapy include alpha- or beta-emitting radioisotopes. An example of a toxin conjugated to an antibody for use in antibody therapy is pseudomonas exotoxin. Examples of drugs that can be used include alkylating agents, platinum drugs, pyrimidine drugs and the like.

However, one of the problems encountered using antibody therapy is that antibodies can target cells expressing an antigen that can be present in both normal and cancerous cells and thus there is a possibility of reacting with normal cells or tissues. Cross reactivity with normal tissues or normal cells can lead to detrimental results. Therefore, tumor specific antigens or antigens overexpressed in tumor cells are generally looked for.

Although murine monoclonal antibodies are easily produced and generally functional, they are recognized as foreign antigens by a human host and human anti-mouse antigens (HAMA) are produced. Because repeated administration of murine antibodies is required, such administration can cause systemic inflammatory effects. To overcome the problems with using murine monoclonal antibodies, one can generate human antibodies directly from humans. However, this process causes ethical problems since one cannot challenge humans with antigens in order to produce antibodies. Furthermore, it is not easy to generate human antibodies against human tissue. Thus, the use of chimeric or humanized antibodies is preferred since they elicit less of a human anti-mouse antibody response.

Chimeric antibodies are antibodies which have variable regions from one species and constant regions from another species. Examples of chimeric antibodies are described in WO88/04936. However, generation of humanized antibodies by introducing human sequences is very difficult and unpredictable. This is due to the significant loss of binding affinity from the grafting of the hypervariable regions and the distortion of the complementary-determining region (CDR) conformation by the human framework. Furthermore, although chimeric antibodies can be generated *in vitro,* functional assays such as CDC assays and ADCC assays cannot inherently predict the *in vivo* capability of a chimeric antibody to destroy or deplete target cells expressing a specific antigen. Thus in many instances, it is not predictable whether a chimeric or humanized antibody can indeed function properly for antibody therapy.

Ferritin is an iron storage protein having a molecular weight of 440,000. It is over expressed in some tumors such as breast cancer (Guner et al., "Cytosol and serum ferritin in breast carcinoma." Cancer Lett, 67(2-3):103-112 (1992); Weinstein et al., "Tissue ferritin concentration and prognosis in carcinoma of the breast." Breast Cancer Res Treat, 14 (3):349-53 (1989)), Hodgkin's lymphoma (Eshhar et al., "Ferritin, a Hodkin;s disease Associated Antigen." PNAS U.S.A.; 71 (10):3956-60 (1974)) and pancreatic cancer (Drysdale et al., "Human isoferritins in normal and disease states." Semin Hematol; 14 (1):71-88(1977) Marcus et al., "Isolation of ferritin from human mammary and pancreatic carcinomas by means of antibody immunoadsorbents." Arch Biochem Biophys: 162 (2):493-501 (1974)).

A radiolabelled polyclonal antiferritin serum obtained by immunization of rabbits with ferritin isolated from the spleen of a Hodgkin's disease patient showed antitumoral activity in patients with Hodgkin's disease (Vriesendorp et al., "Radiolabelled immunoglobulin therapy in patients with Hodgkin's disease." Cancer Biother Radiopharm: 15 (5):431-45 (2000). However, polyclonal antibodies have several drawbacks in comparison to monoclonal antibodies such as the limited batch size. Indeed, Vriesendorp and colleagues noticed differences in response rates in two trials on Hodgkin's lymphoma patients when they used two different batches of the polyclonal antiferritin antibody (Vriesendorp et al., "Review of five consecutive studies of radiolabeled immunoglobulin therapy in Hodgkin's disease." Cancer Res; 55 (23 Suppl): 5888s92s (1995). Thus, monoclonal antibodies are preferable to use in therapeutic applications in lieu of polyclonal antibodies for many reasons.

U.S. Patent 7,153,506 describes the use of murine anti-ferritin monoclonal antibodies to treat some forms of cancer. However, as set forth above there are problems associated with treating humans with murine monoclonal antibodies (such as HAMA production).

WO01/52889 also describes monoclonal and polyclonal antibodies binding an epitope common to acidic and basic ferritins, and their use in treating tumor cells overexpressing a gene of the myc family. However, WO01/52889 does not identify the structure and sequence of the domain of fixation of said monoclonal and polyclonal antibodies to their corresponding epitope(s).

Sabbah et al. "In vitro and in vivo comparison of DTPA- and DOTA-conjugated antiferritin monoclonal antibody for imaging and therapy of pancreatic cancer" (Nuclear Medicine and Biology, 2077, vol. 34, n°3, pp 293-304) present results obtained from the preclinical development of a monoclonal IgG1 antiferritin antibody named AMB8LK, conjugated to bifunctional chelating ligands and radiolabelled. However, Sabbah et al. do not identify the structure and sequence of the domain of fixation of said monoclonal antibody to its epitope.

Pancreatic cancer has a very poor prognosis with less than a 5% survival rate at five years. Neither external beam radiation nor chemotherapy, alone or in combination have given encouraging results so far. Up to 95% of the cases of pancreatic cancer arise from the exocrine part of the pancreas, which is that part of the pancreas which produces enzymes in acinar cells. Most pancreatic cancer is found in the ductal cells. Therefore, the term adenocarcinoma (duct-like) has been assigned to the most common cause of pancreatic cancers. More than 60,000 Europeans are diagnosed with adenocarcinoma each year. The survival period from the time of diagnosis until the time of death is the worst for any cancers and is generally about $3\frac{1}{2}$ to 6 months. Surgical removal of the tumor is an option, but only about 15% to 20% of patients with pancreatic cancer are eligible for surgery.

Besides pancreatic cancer, other cancers such as Hodgkins lymphoma, breast cancer and hepatocellular carcinomas are also cancers that have high death rates and, for some of them, limited opportunities for surgical removal. For instance in hepatocellular carcinoma if the tumor is less than 5 cm, exists on a sole lobe in the liver and there is no invasion of liver vasculature by the carcinoma, the tumor can be removed surgically. However, surgical removal is impossible in many cases due to the non-diagnosed progression of the disease.

There is a need for a method of treating the above quoted cancers that does not involved surgical intervention and that would reduce the secondary effects associated with chemotherapy.

Thus, the present application discloses pharmaceutical compositions containing a chimeric or humanized anti-ferritin antibody or fragments thereof as well as antibody-like molecules that can bind human acidic and basic ferritin for treating certain cancers.

The present application also discloses pharmaceutical compositions containing a chimeric or humanized anti-ferritin antibody or fragments thereof as well as antibody-like molecules that can treat pancreatic cancer, breast cancer, hepatocellular carcinomas, Kaposi's sarcoma and Hodgkin's lymphoma.

The present application also discloses chimeric or humanized monoclonal antibodies which are specific to human acidic and basic ferritin.

The present application also discloses chimeric or humanized antibodies specific to human acidic and basic ferritin that bind to human ferritin.

The present application also discloses methods of treating cancers selected from the group of pancreatic cancer, breast cancer, hepatocellular carcinomas, Kaposi's sarcoma and Hodgkin's lymphoma by administering a chimeric or humanized anti-ferritin antibody or fragments thereof as well as antibody-like molecules.

The present application also discloses methods of treating cancers selected from the group of pancreatic cancer, breast cancer, hepatocellular carcinomas, Kaposi's sarcoma and Hodgkin's lymphoma by administering a radiolabelled chimeric or humanized anti-ferritin antibody or fragments thereof as well as antibody-like molecules conjugated to a chelator.

The present application also discloses a method for delivering humanized or chimeric anti-ferritin antibodies to cancerous lymph cells, pancreatic cells, lymphatic endothelium cells, and liver cells.

The present application also discloses the use of a chimeric or humanized anti-ferritin antibody or fragments thereof as well as antibody-like molecules for the preparation of a drug to treat certain cancers such as pancreatic cancer, breast cancer, hepatocellular carcinomas, Kaposi's sarcoma and Hodgkin's lymphoma.

The present application also discloses the provision of nucleic acid sequences that can be placed in vectors to produce all or part of a chimeric or humanized anti-ferritin antibody or fragments thereof as well as antibody-like molecules of the present invention via the production in cells.

These and other objects of the present application will become apparent from the following description, examples or preferred embodiments.

## SUMMARY


In one aspect, the present application discloses a, single chain, polypeptide comprising, from N-terminal to C terminal, SEQ ID NO:30, SEQ ID NO:28 and SEQ ID NO:26 (to form a VH domain). In another aspect, this polypeptide also comprises, in the C-terminal of SEQ ID NO:26, a CH1 domain such as SEQ ID NO:6, as well as a Fc region such as SEQ ID NO:8, SEQ ID NO:10 or SEQ ID NO:18, to form a heavy chain. In another aspect, the present application describes a single chain polypeptide comprising, from N-terminal to C terminal, SEQ ID NO:20, SEQ ID NO:22 and SEQ ID NO:24, to form a VL domain. In another aspect, this single polypeptide also comprises, in the C-terminal of SEQ ID NO:24, a kappa or a lambda chain such as SEQ ID NO:12 to form a light chain.

In another aspect, the application discloses a molecule, optionally binding both human acidic and basic ferritin, comprising a light chain as defined herein, a heavy chain as defined herein or any combination thereof. In a yet another aspect, said molecule is a chimeric anti-ferritin monoclonal antibody that binds both human acidic and basic ferritin. In a yet another aspect, the chimeric anti-ferritin monoclonal antibody comprises the sequences of SEQ ID Nos. 2, 4, 6, 8 and 12, or SEQ ID Nos.:2, 4, 6, 10 and 12.

In another aspect the application relates to a chimeric monoclonal anti-ferritin antibody that binds both human acidic and basic ferritin comprising the amino acid sequences of SEQ ID Nos. 2, 4, 6, 12 and 18.

More specifically, the present invention relates to a chimeric anti-ferritin monoclonal antibody, that binds both human acidic and basic ferritin, comprising: (a) two heavy chain polypeptides, each comprising the VH domain defined in SEQ ID NO:4; and (b) two light chain polypeptides, each comprising the VL domain as defined in SEQ ID NO:2.

According to a particular embodiment, each of said heavy chains further comprises a CH1 constant region, optionally comprising a human Fc region or a murine Fc region, and/or a signal peptide in said chimeric anti-ferritin monoclonal antibody. Within said particular embodiment, said signal peptide may comprise the amino acid sequence set forth in SEQ ID NO:16.

In another particular embodiment, each of said light chains further comprises a constant kappa or lambda region and/or a signal peptide in said chimeric anti-ferritin monoclonal antibody. Within said another particular embodiment, said constant kappa or lambda region may comprise the amino acid sequence set forth in SEQ ID NO:12 or said signal peptide may comprise the amino acid sequence set forth in SEQ ID NO:14.

The chimeric anti-ferritin monoclonal antibody of the invention may comprise as a light chain, from the N-terminal part to the C-terminal part, SEQ ID NO:2 and SEQ ID NO:12, and as a heavy chain, from the N-terminal part to the C-terminal part, SEQ ID NO: 4, SEQ ID NO:6 and a human Fc region.

In another aspect, the present invention relates to a functional fragment of the antibody of the invention, wherein said functional fragment is a Fv fragment or a Fab fragment and wherein said functional fragment specifically binds both human acid and basic ferritins. In a particular embodiment, said fragment is linked to a ligand, such as cytokine, a receptor or any protein of interest.

The invention also relates to a bispecific or trispecific monoclonal antibody comprising at least one Fab fragment as defined above, wherein at least one Fab fragment consists of a polypeptide consisting from the N-terminal to the C-terminal of SEQ ID NO:2 directly linked to SEQ ID NO:12 and a polypeptide consisting from the N-terminal to the C-terminal of SEQ ID NO:4 directly linked to SEQ ID NO:6.

The invention also relates to a scFv molecule consisting of a VH domain as defined in SEQ ID NO:4, linked to a VL domain as defined in SEQ ID NO:2, wherein said scFv molecule binds both human acidic and basic ferritins. The invention also relates to a diabody molecule comprising at least a VH domain as defined in SEQ ID NO:4 and at least a VL domain as defined in SEQ ID NO:2, wherein said diabody molecule binds both human acidic and basic ferritins.

A murine anti-ferritin monoclonal antibody that binds both human acidic and basic ferritin comprising as a light chain, from the N-terminal part to the C-terminal part, SEQ ID NO:2 and SEQ ID NO:12, and as a heavy chain, from the N-terminal part to the C-terminal part, SEQ ID NO:4, SEQ ID NO:6 and a murine Fc region, is also part of the invention.

The invention also encompasses a molecule selected from the group consisting of a chimeric anti-ferritin monoclonal antibody, a functional fragment, a bispecific monoclonal antibody, a trispecific monoclonal antibody and a scFv molecule, a Bis-scFv comprising at least one scFv according to definition provided above and a diabody according to the definitions provided above, further comprising a radioisotope conjugated thereto. In a particular embodiment, said radioisotope is an alpha-, beta-, gamma-, beta-gamma- or alpha-beta-emitting radioisotope.

The invention also encompasses a molecule selected from the group consisting of a chimeric anti-ferritin monoclonal antibody, a functional fragment, a bispecific monoclonal antibody, a trispecific monoclonal antibody and a scFv molecule to the definitions provided above, a Bis-scFv comprising at least one scFv according to the definitions provided above and a diabody according to the definitions provided above, further comprising a drug conjugated thereto. In a particular embodiment, said drug is a pyrimidine drug.

A pharmaceutical composition comprising a molecule according to the definition given herein, especially a chimeric or humanized monoclonal anti-ferritin antibody that binds both human acidic and basic ferritin, fragment thereof or antibody-like molecules as disclosed in the present application that binds both human acidic and basic ferritin as disclosed herein, and especially a chimeric anti-ferritin monoclonal antibody comprising the amino acid sequences of SEQ ID Nos. 2, 4, 6, 8 and 12 or SEQ ID Nos. 2, 4, 6, 10 and 12, said composition comprising further a pharmaceutically acceptable vehicle and optionally a pyrimidine drug, is yet another aspect of the invention.

The invention also encompasses a pharmaceutical composition comprising a molecule selected from the group consisting of a chimeric or human anti-ferritin monoclonal antibody of the invention as defined above, a functional fragment according to the definitions provided above, a bispecific or trispecific monoclonal antibody according to the definitions provided above, a scFv molecule according to the definitions provided above, a Bis-scFv comprising a scFv molecule according to the definitions provided above and a diabody according to the definitions provided above, and a pharmaceutically acceptable vehicle.

In yet another aspect a method of delivering a drug, radioactive materials, toxins, immune killer cells or combinations thereof to cells containing basic and acidic ferritin, said method comprising administering a composition comprising a heavy chain, a light chain, any combination thereof, a chimeric or humanized antibody or a fragment thereof or an antibody-like molecule of the invention, and a composition selected from the group of a drug, radioactive materials, toxins, immune killer cells and combinations thereof wherein said composition is in a pharmaceutically acceptable carrier is disclosed.

A method of treating a cancer selected from the group of pancreatic cancer, Hodgkin's lymphoma, Kaposi's sarcoma and hepatocellular carcinoma said method comprising administering to a mammal in need of such treatment a composition comprising a heavy chain, a light chain, any combination thereof, a chimeric or humanized antibody, a fragment thereof or an antibody-like molecule of the application, especially a molecule comprising SEQ ID Nos: 2, 4, 6, 8 and 12 or SEQ ID Nos. 2, 4, 6, 10 and 12, and optionally a composition selected from the group of a drug, radioactive isotopes, toxins, immune killer cells and combinations thereof wherein said composition is in a pharmaceutically acceptable carrier is yet another aspect of the present disclosure.

Is also part of the invention the composition defined above, in a pharmaceutically acceptable carrier, wherein a drug, radioactive materials, toxins, immune killer cells or combinations thereof is attached through a chelating agent or a linker to the chimeric anti-ferritin monoclonal antibody, functional fragment, bispecific or trispecific monoclonal antibody, scFv molecule, Bis-scFv or diabody, for use as a therapeutic agent.

Is also part of the invention the composition defined above, in a pharmaceutically acceptable carrier for use in combination with a composition selected from the group of a drug, radioactive materials, toxins, immune killer cells and combinations thereof, in the treatment of a cancer selected from the group consisting of pancreatic cancer, Hodgkins lymphoma, Kaposi sarcoma and hepatocellular carcinoma.A nucleic acid sequence comprising SEQ ID Nos: 1 and 11 or a nucleic acid comprising SEQ ID Nos. 3, 5 and 7 or SEQ ID Nos 3, 5 and 9 or SEQ ID Nos 3, 5 and 17 , or a nucleic acid sequence comprising SEQ ID NOs. 1, 3, 5, 7 and 11 or SEQ ID Nos 1, 3, 5, 9 and 11, or a nucleic acid comprising SEQ ID Nos: 1, 11, 13 or a nucleic acid comprising SEQ ID Nos 3, 5, 7 and 15, or a nucleic acid comprising SEQ ID Nos. 3, 5, 9 and 15 or a nucleic acid comprising SEQ ID Nos. 3, 5, 15 and 17, as well as vectors containing at least one of these nucleic acid sequences and cells transformed with at least one of these vectors are also described. A nucleic acid comprising SEQ ID NO:19, 21 and 23 and optionally SEQ ID NO:11 as well as a nucleic acid comprising SEQ ID NO:29, 27 and 25 and optionally SEQ ID NO:5, and further optionally SEQ ID NO:7, NO:9 or NO:17 are also part of the present application.

The invention also encompasses a vector comprising a nucleic acid encoding a heavy chain as defined above and a nucleic acid encoding a light chain as defined above, and optionally comprising a promoter for baculovirus expression or a eukaryotic promoter. According to a particular embodiment of said vector, said nucleic acid encoding the heavy chain comprises SEQ ID NO:3 and wherein said nucleic acid encoding the light chain comprises SEQ ID NO:1.

The invention also encompasses cells transformed with a vector as defined above, wherein said cells are selected from the group consisting of CHO cells, E. *coli,* yeast cells, VERO cells, HELA cells, COS cells, CR cells: 1650, W138, BHK, HepG2, 3T3, A549, PC12, K562, 293 cells, Sf9 cells and Cv1 cells.

The invention also encompasses cells transformed with a vector comprising a nucleic acid encoding a heavy chain as defined above and with a vector comprising a nucleic acid encoding a light chain as defined above, in particular selected from the group consisting of CHO cells, *E. coli,* yeast cells, VERO cells, HELA cells, COS cells, CR cells: 1650, W138, BHK, HepG2, 3T3, A549, PC12, K562, 293 cells, Sf9 cells and Cv1 cells.

According to a particular embodiment, said nucleic acid encoding the heavy chain comprises SEQ ID NO:3, and said nucleic acid encoding the light chain comprises SEQ ID NO:1 in said cells.

**BRIEF DESCRIPTION OF THE DRAWINGS**

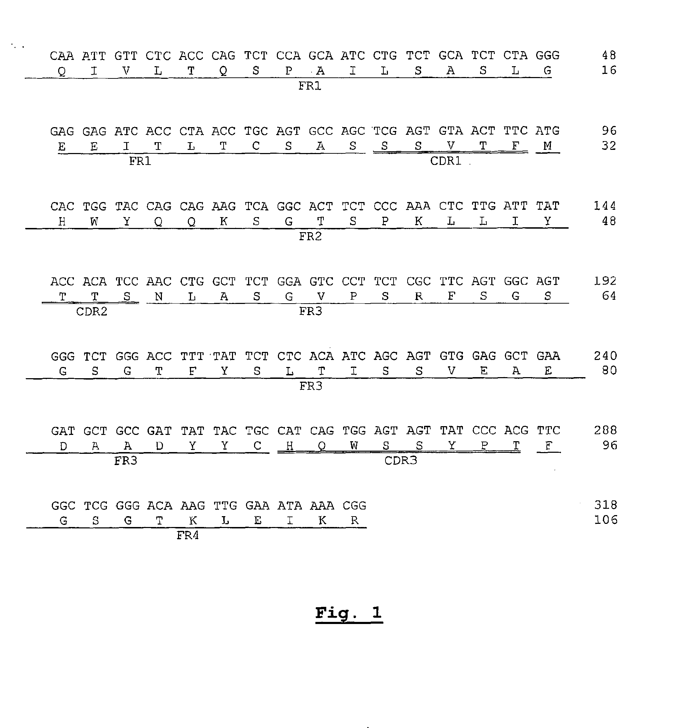

Fig. 1 is the VL nucleic acid (SEQ ID NO: 1) and amino acid sequence (SEQ ID NO:2) from clone AMB8LK. The VL domain is composed of the FR1 region (amino acids 1-26), the CDR1 region (amino acids 27-31), the FR2 region (amino acids 32-48), the CDR2 region (amino acids 49-51), the FR3 region (amino acids 52-87), the CDR3 region (amino acids 88-95) and the FR4/J region (amino acids 96-106).

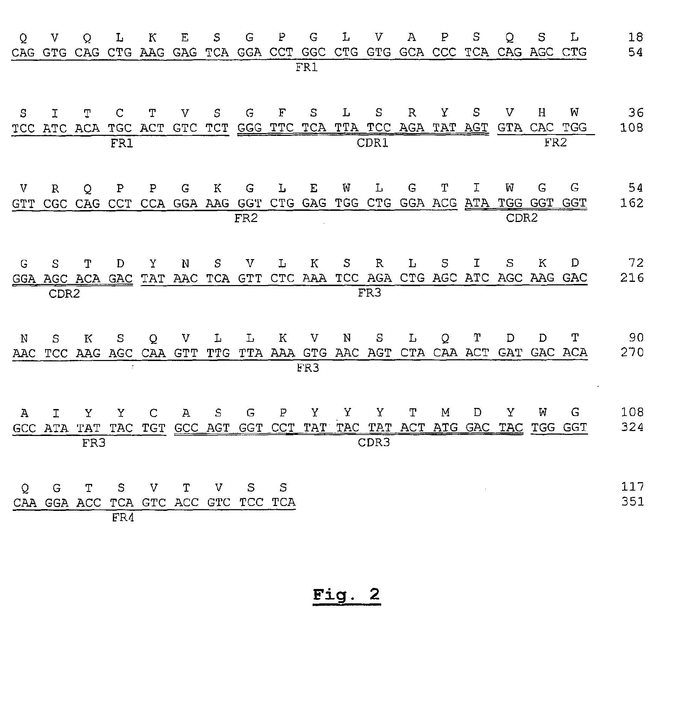

Fig. 2 is the VH nucleic acid (SEQ ID NO: 3) and amino acid sequence (SEQ ID NO:4) from clone AMB8LK. The VH domain is composed of the FR1 region (amino acids 1-25), the CDR1 region (amino acids 26-33), the FR2 region (amino acids 34-50), the CDR2 region (amino acids 51-58), the FR3 region (amino acids 59-95), the CDR3 region (amino acids 96-106) and the FR4/J region (amino acids 107-117).

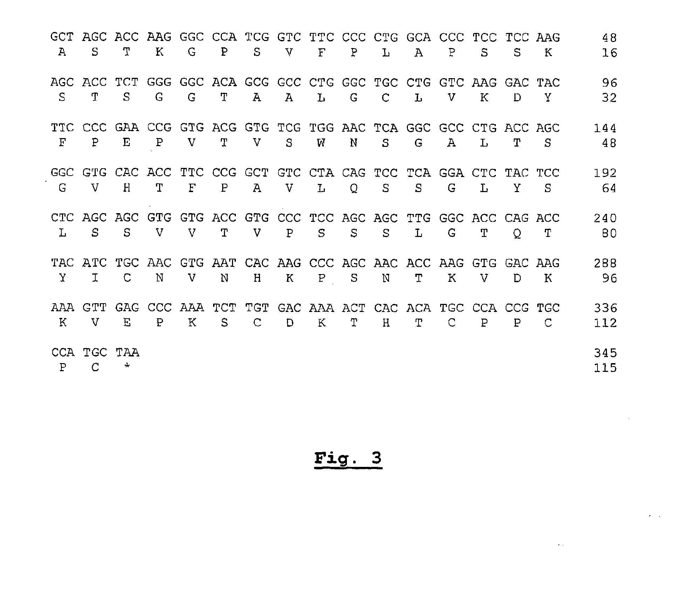

Fig. 3 is the CH1 nucleic acid (SEQ ID NO: 5) for the Fab'2-3 cyst construct and amino acid sequence (SEQ ID NO:6) for the Fab'2-3 cyst construct. The CH1 was cloned from a human constant gamma 1 nucleic acid sequence, supplemented by an additional cystein and a stop codon.

Fig. 4 is the nucleic acid sequence of the human gamma 1 constant region (SEQ ID NO: 7) and the amino acid sequence of the human gamma 1 constant region (SEQ ID No; 8) (Genbank accession number BC073782). The second TCC codon was modified to AGC (underlined) to create a *Nhe*I cloning site.

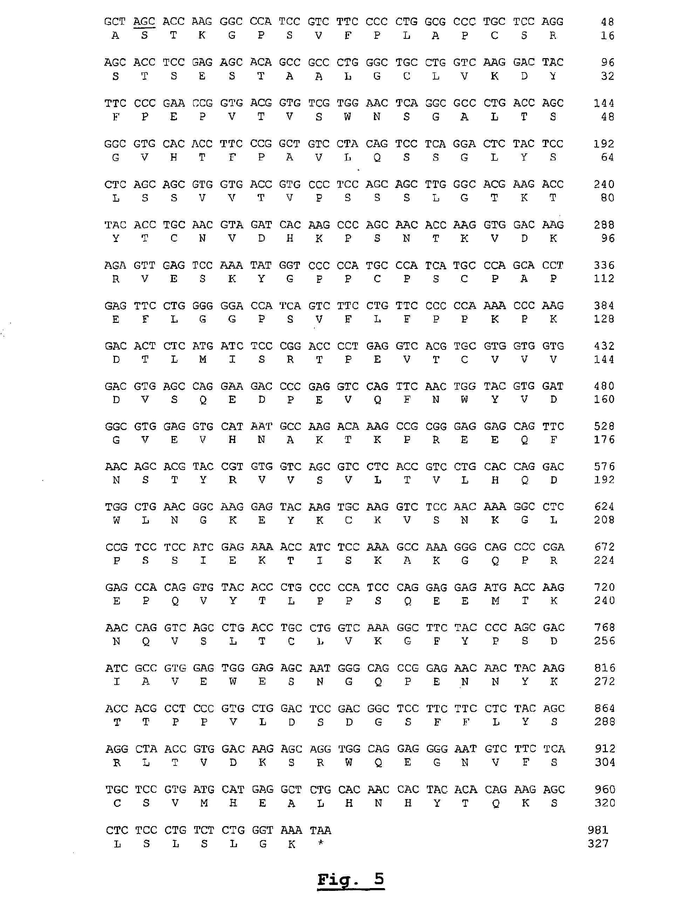

Fig. 5 is the nucleic acid sequence of the human gamma 4 constant region (SEQ ID NO: 9) and the amino acid sequence of the human gamma 4 constant region (SEQ ID No: 10) (Genbank accession number BC025985). The second TCC codon was modified to AGC (underlined) to create a *Nhe*I cloning site.

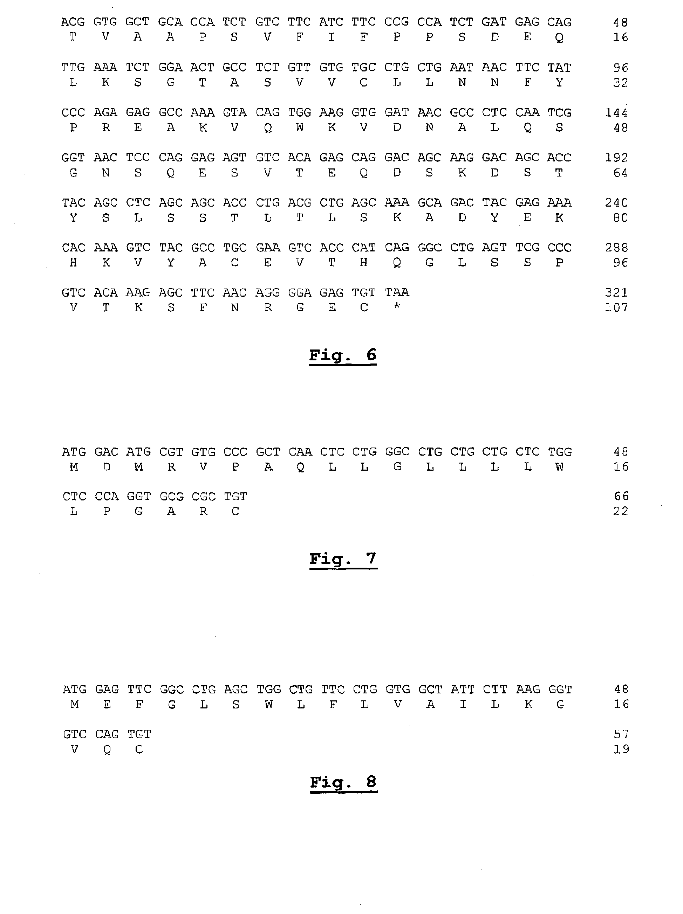

Fig. 6 is the nucleic acid sequence of the human kappa constant region (SEQ ID NO: 11) and the amino acid sequence of the human kappa constant region (SEQ ID No; 12).

Fig. 7 is the nucleic acid sequence of the human VL signal peptide sequence (SEQ ID NO: 13) and the amino acid sequence of the human VL signal peptide sequence (SEQ ID NO: 14).

Fig. 8 is the nucleic acid sequence of the human VH signal peptide sequence (SEQ ID NO: 15) and the amino acid sequence of the human VH signal peptide sequence (SEQ ID NO: 16).

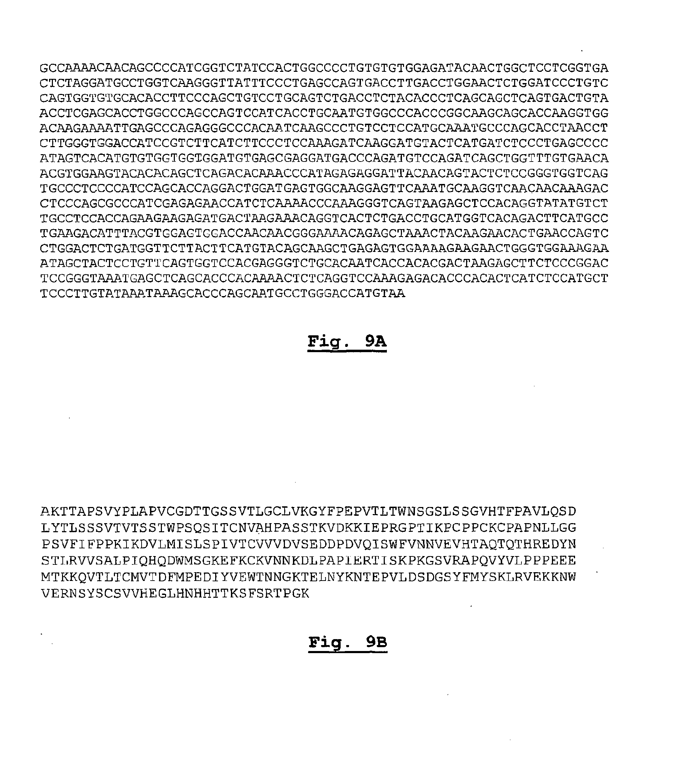

Fig. 9A is the nucleic acid sequence of the constant region from murine $\gamma$ 2a (SEQ ID NO: .17)

Fig. 9B is the amino acid sequence of the constant region from murine $\gamma$ 2a (SEQ ID NO: 18).

Fig. 10 is the chemical structure of the chelates used in the examples. A: *p*SCN-Bz-DTPA: 2-(4-isothiocyanatobenzyl)-diethylenetriaminepentaacetic acid. B: *p*SCN-Bz-CHX-A''-DTPA: (*R*)-2-amino-3-(4-isothiocyanatophenyl)propyl]-trans-(*S,S*)-cyclohexane-1,2-diamine-pentaacetic acid; C: *p*SCN-Bz-DOTA: 2-(4,isothiocyanatobenzyl)-1,4,7,10-tetraazacyclodedecane-1,4,7,10-tetraacetic acid.

Fig. 11 is a graph showing the immunoreactivity of the cold conjugated antibody by ELISA. After conjugation of about 3 chelates per antibody the assessment of the immunoreactivity of the immunoconjugates was performed by ELISA. Pure ferritin was immobilized at 5 $\mu$g/ml in PBS, then incubated with various concentrations of the immunoconjugates. The control was the unconjugated AMB8LK.

Figs. 12A, B and C are graphs showing the biodistribution in CAPAN-1 tumor bearing mice of $^{111}$In-DTPA-AMB8LK

and $^{111}$IN-DOTA-AMB8LK. 0.2 MBq of indium-labelled Bz-DTPA-AMB8LK or Bz-DOTA-AMB8LK were intravenously injected in CAPAN-1 tumor bearing mice. Animals were sacrificed at different time points and the organs were collected for activity counting. Data are expressed as percentage of the injected dose per organ (or per gram of tissue for the tumor, blood, muscle and bone. Error bars represent SD, n=4 per group. The abbreviation Intest stands for intestine. Graph A is the distribution 24 hours after injection. Graph B is the distribution 48 hours after injection. Graph C is the distribution 72 hours after injection.

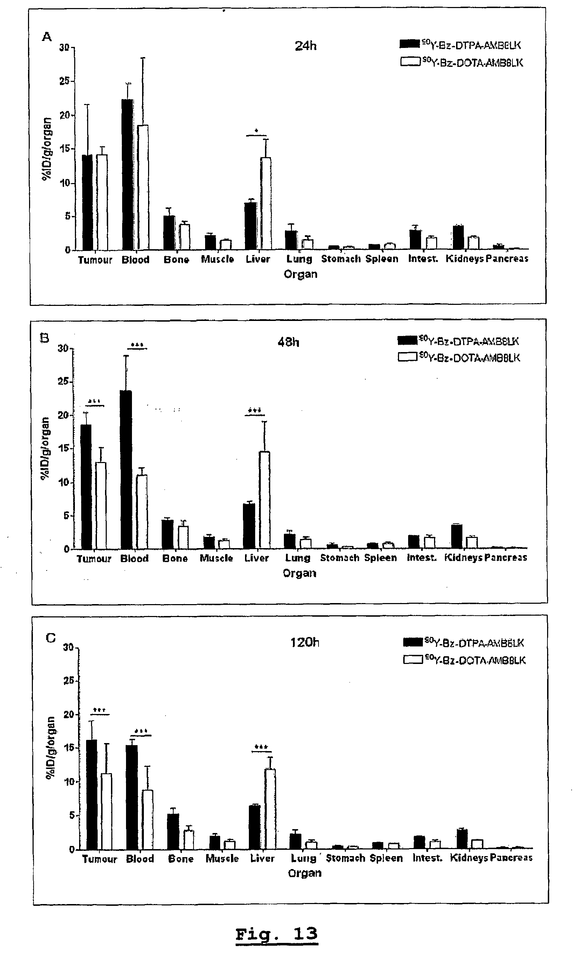

Figs. 13A, B and C are graphs showing the biodistribution in CAPAN-1 tumor bearing mice of $^{90}$Y-Bz-DTPA-AMB8LK and $^{90}$Y-Bz-DOTA-AMB8LK. 0.4 MBq of yttrium-labelled Bz-DTPA-AMB8LK or Bz-DOTA-AMB8LK were intravenously injected in CAPAN-1 tumor bearing mice. Animals were sacrificed at different time points and the organs were collected for activity counting. Data are expressed as percentage of the injected dose per organ (or per gram of tissue for the tumor, blood, muscle and bone). Error bars represent SD, n=4 per group. The abbreviation Intest stands for intestine. Graph A is the distribution 24 hours after injection. Graph B is the distribution 48 hours after injection. Graph C is the distribution 120 hours after injection.

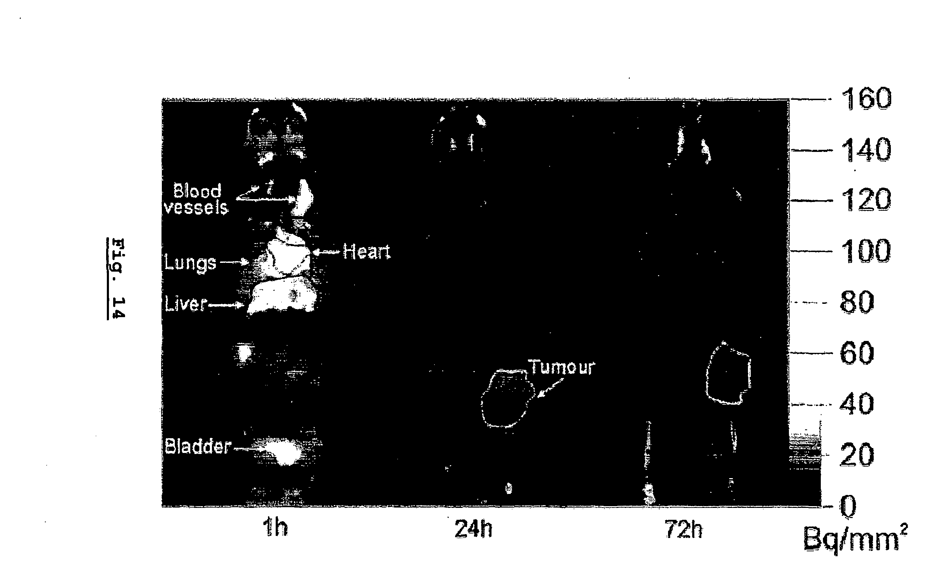

Fig. 14 are three images of a CAPAN-1 tumor bearing mouse injected with $^{111}$In-DOTA-AMB8LK. A CAPAN-1 tumor bearing nude mouse was intravenously injected with 20 MBq of $^{111}$In-DTPA-AMB8LK then imaged at different time points. This figure shows the scans done 1, 24 and 72 hours after injection, The same scale of radioactivity levels was applied to the three images but taken into account the decay of the indium, the radioactivity level in the tumor after 72 hours is superior to that after 24 hours.

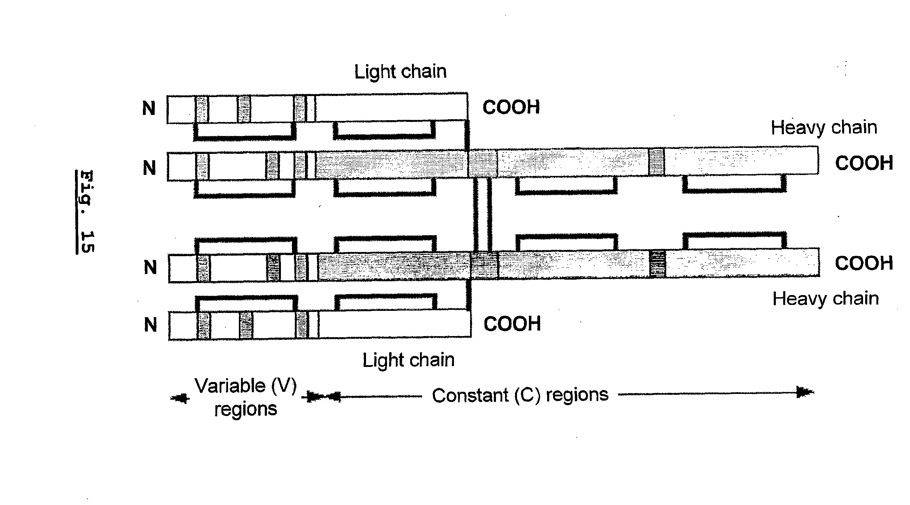

Fig. 15 is a diagram of the monoclonal antibody of the present invention.

BRIEF DESCRIPTION OF THE SEQUENCES

Sequences (SEQ ID) used in the present application are the following:

| Name | Nucleotide SEQ ID | Protein SEQ ID |
|---|---|---|
| VL domain from clone AMB8LK | 1 | 2 |
| VH domain from clone AMB8LK | 3 | 4 |
| CH1 domain (Fab'2-3 cyst construct) | 5 | 6 |
| human gamma 1 constant region | 7 | 8 |
| human gamma 4 constant region | 9 | 10 |
| human kappa constant region | 11 | 12 |
| VL signal peptide | 13 | 14 |
| VH signal peptide | 15 | 16 |
| constant region from murine $\gamma$ 2a | 17 | 18 |
| VL domain CDR1 | 19 | 20 |
| VL domain CDR2 | 21 | 22 |
| VL domain CDR3 | 23 | 24 |
| VH domain CDR3 | 25 | 26 |
| VH domain CDR2 | 27 | 28 |
| VH domain CDR1 | 29 | 30 |
| VH domain FR1 | 31 | 32 |
| VH domain FR2 | 33 | 34 |
| VH domain FR3 | 35 | 36 |
| VH domain FR4 | 37 | 38 |
| VL domain FR1 | 39 | 40 |

(continued)

| Name | Nucleotide SEQ ID | Protein SEQ ID |
|---|---|---|
| VL domain FR2 | 41 | 42 |
| VL domain FR3 | 43 | 44 |
| VL domain FR4 | 45 | 46 |

## DESCRIPTION OF THE PREFERRED EMBODIMENTS

As used herein the term "consisting essentially of" when used in connection with nucleic acids or amino acids means that other minor ingredients or molecules may be present with the amino acid or nucleic acid sequences. The nucleic acid sequence has the exact same length as indicated in the sequence identification number, but may have 3 to 12 extra or less nucleotides at the N-and C- terminals. Likewise, the amino acid sequence has the exact same length as indicated in the sequence identification number but from 1 to 4 extra or less amino acids may be added or deleted at the N- or C- terminals. These extra or deleted amino acids do not modify the binding and/or the activity of the chimeric monoclonal antibody to ferritin.

As used herein the term "chelating agent" means any chemical substance that complexes with a metal to form a chelate. Chelating agents are organic compounds that are capable of forming coordinate or ionic bonds with metals through two or more atoms of the organic compound.

The term "mammal" encompasses any of various warm-blooded vertebrate animals of the class Mammalia, including humans, characterized by a covering of hair on the skin and, in the female, milk-producing mammary glands for nourishing the young. In a particular embodiment, the disclosure relates to a non-human mammal.

The term "chimeric antibody" and "humanized antibody" refer to an antibody whose regions are derived from two different species, generally murine and human. The term "chimeric antibody" is specifically used to refer to a variable region (composed of a VL and VH and responsible for the binding of the antigen) of a first species, such as a mouse with the constant region of a second species, such as human. The expression "humanized antibody" refers rather to the grafting of complementary determining-regions (CDR) from a first species, such as mouse, into human framework regions (FR).

The term "monoclonal antibody" refers to an antigen-binding protein having four-polypeptide chains consisting of two heavy and two light chains. The chains, that are stabilized by interchain disulfide bonds, have the ability to specifically bind antigen. Both heavy and light chains are folded into domains, which is the globular region of the heavy or light chain containing peptide loops. The domains are either "constant" or "variable" depending upon the amount of variation in the sequence. Constant domains on the light chain are referred to as light chain constant regions or CL. Constant domains on the heavy chain are referred to as heavy chain constant domains or CH. Variable domains on the light chain are referred to as light chain variable regions or VL domain, while heavy domains on the variable chains are referred to as heavy chain variable regions or VH domain. Each variable domain (VH or VL) consists of CDR (complementary determining region) and FR (framework region), that are alternatively linked together *i.e.,* from N-terminal to C-terminal: FR1-CDR1-FR2-CDR2-FR3-CDR3-FR4.

The expression "monoclonal antibody" encompasses:

(1) monospecific antibodies *i.e.,* molecules wherein the two antigen binding sites (domains formed by the interaction of the VH and VL regions as defined herein, and interacting with the antigen) recognize and bind the same antigen (*e.g.* , an antigen common to human acidic and basic ferritins). In this aspect, the two heavy chains and two light chains as defined herein (or at least the two VH and two VL as defined herein) have the same amino acid sequence;
(2) trifunctional antibodies *i.e.,* bispecific molecules as disclosed hereinafter and further having an Fc region (CH2 and CH3 domains) of any origin, particularly of human origin.

The term "antibody-like molecule" refers to a molecule having all or part of the variable heavy and light domains of the AMB8LK antibody, and not having the conventional structure of a four-chain antibody, but conserving the capacity to interact and bind with the antigen (ferritin). In a particular embodiment, the antibody-like molecules described herein comprise the CDR1, CDR2 and CDR3 regions of the VL and/or VH domains of the AMB8LK antibody. This encompasses:

(1) scFv, *i.e.,* a VH domain as defined herein genetically associated (optionally via a linker) to a VL domain as defined herein, as well as molecules comprising at least one scFv, such as Bis ScFv molecules (two ScFv having same or different antigen binding linked together (optionally via a linker));
(2) diabody molecules *i.e.,* the heavy chain variable domain derived from a first antibody (a first VH domain (VH1) such as one defined herein) connected to the light chain variable domain derived from a second antibody (VL2) on the same polypeptide chain (VH1-VL2) connected by a peptide linker that is too short to allow pairing between the two domains on the same chain, interacting with the heavy chain variable domain of derived from a second antibody (VH2) connected to the light chain variable domain derived from a first antibody (a first VL domain (VL1) such as one defined herein) on the same polypeptide chain (VH2-VL1), wherein VL1 and VH1 form a first antigen-binding site (recognizing and/or binding an antigen common to human acidic and basic ferritin) and VL2 and VH2 form a second antigen binding site (recognizing and/or binding a similar or a different antigen and/or epitope from the first binding antigen binding site);

(3) bispecific molecules *i.e.,* that the two antigen binding sites of a $Fab_2$ fragment (variable and CH1 domains of light and heavy chains) interact with different antigens (or epitopes). In this aspect, the two heavy chains (or the two VH domains) have a different amino acid sequence, and/or the two light chains (or the two VL domains) have a different amino acid sequence. In a bispecific molecules of the invention, at least one of the two antigen binding sites is a ferritin binding site consisting of a VL or VH domains as defined herein, such as respectively SEQ ID NO:2 and SEQ ID NO:4 or variants thereof;

(4) trispecific molecules *i.e.,* that the two antigen binding sites of a $Fab_3$ fragment (variable and CH1 domains of light and heavy chains) interact with different antigens (or epitopes). In this aspect, the three heavy chains (or at the three VH domains) have a different amino acid sequence, and/or the three light chains (or the three VL domains) have a different amino acid sequence. In a tri-specific molecules of the invention, at least one of the three antigen binding sites is a ferritin binding site consisting of a VL or VH domains as defined herein, such as respectively SEQ ID NO:2 and SEQ ID NO:4 or variants thereof;

(5) $V_{HH}$ (VH domain devoid of light chain) *i.e.,* a VH domain as defined herein which has the capacity to interact as such with the antigen, without the presence of a variable light domain (VL). These $V_{HH}$ can be obtained by recombinant techniques or by purification from animal of the camelid family or from sharks.

By "constant region" is meant the CH1, CH2 and CH3 domains (CH2 and CH3 forming the Fc region). In the monoclonal antibodies of the present disclosure, the murine constant CH1 domain and murine constant CH2 and CH3 domains are replaced, independently from one another, with human constant CH1 and human constant CH2 and CH3 regions. Therefore, in one embodiment, the CH2 and CH3 domains (Fc region) of a murine monoclonal antibody are replaced by a CH2 and CH3 regions of human origin. The human Fc region may be a mu, gamma, alpha, delta or epsilon chain, optionally derived from human immunoglobulins. In a particular embodiment, the human Fc region is selected from IgG1 and IgG4. In another embodiment, the CH1 domain of a monoclonal antibody is replaced by a human CH1 region, such as kappa or lambda chains, optionally derived from human immunoglobulins.

By "functional fragment" is meant a monoclonal antibody fragment, which retains its binding properties to specifically bind to both human acidic and basic ferritin. In another aspect "functional fragment" encompasses a monoclonal antibody that exhibits the same or substantially the same binding affinity and avidity as the non-fragmented chimeric anti-ferritin monoclonal antibody or parent monoclonal antibody. The binding affinity of the monoclonal antibody functional fragment should not be less than 10% of the parent antibody. In another aspect the binding affinity of the monoclonal antibody fragment should not be less than 25% of the parent antibody. In another aspect binding affinity of the monoclonal antibody fragment should not be less than 30% or not be less than 50% of the parent antibody. Methods for measuring binding affinity are well known in the art and include, for example competition assays, Scatchard analysis and half-maximal binding assays.

By the term "variant" when referring to the sequence variants means sequences having a degree of similarity of of at least 90%, 95% or 98% similarity with SEQ ID Nos: 2, 4, 6, 8, 10, 12, 16, 18, 20, 22, 24, 26, 28, 30, 32, 34, 36, 38, 40, 42, 44 and 46, using sensitive and rapid sequence comparison programs such as BLAST, FASTA, GAP, BESTFIT, clustalW or any other relevant program.

Clearly, the amino acid sequences with a specific identity defined above have a majority of conservative amino acid substitutions. Conservative amino acid substitutions include amino acid substitutions of the same class. These classes comprise, for example, amino acids having uncharged polar side chains, such as Asn, Gln, Ser, Thr or Tyr; amino acids containing basic side chains, such as His, Lys or Arg; amino acids containing acidic side chains, such as Glu or Asp and amino acids containing non-polar side chains, such as Ala, Gly, Leu, Val, Ile, Phe, Cys or Trp.

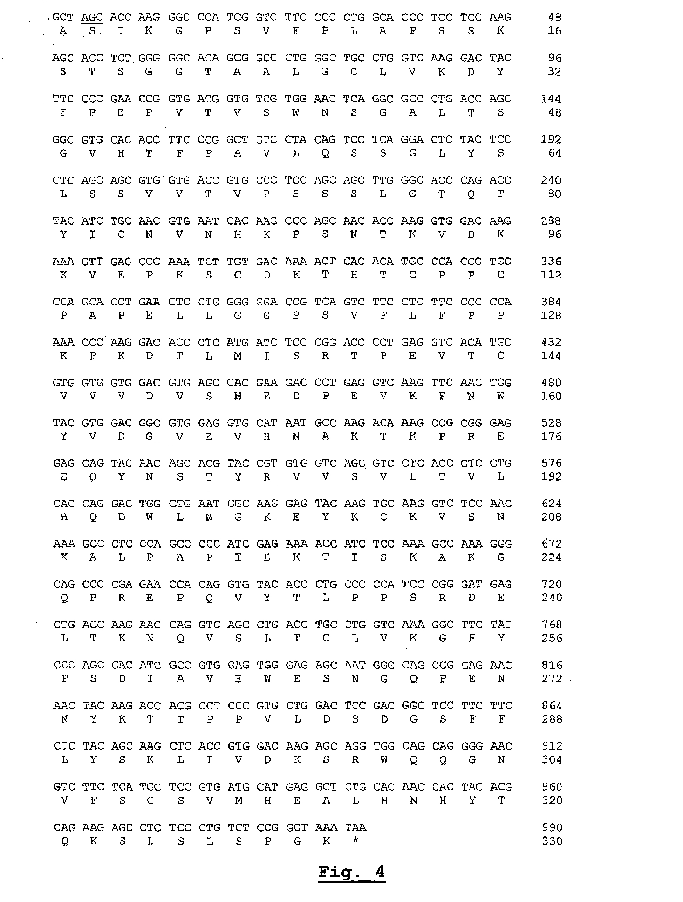

For example, with respect to Figure 4, the Asn at amino acid position 42 can be substituted with Gln, Ser, Thr or Tyr; the Lys at amino acid position 11 can be modified to His or Arg; the Ala at amino acid position 7 can be replaced by Gly, Leu, Val, Ile, Phe, Cys or Trp. Likewise, conservative substitutions can be made to any of the amino acids sequences of the present invention in the same manner. It will be appreciated that the person skilled in the art would ensure that once these substitutions are made to the protein sequence or sequences, human ferritin binding activity is retained. This can be accomplished by using the RIA and ELISA assays described in the examples.

The term "epitope" refers to the site of the antigen to which the antibody specifically binds. An epitope has at least 3 to 15 amino acids in a unique conformation (linear or conformational epitope). X-ray crystallography, 2-dimensional nuclear magnetic resonance or epitope mapping can be used as methods for determining spatial conformation of epitopes.

Antibodies that recognize the same epitope can be identified by a competitive binding assay, which is known in the art. Examples of competitive binding assays include RIA and ELISA.

The term "effective dosage" or "effective dose" means the amount sufficient to achieve or at least achieve partially the desired effect. The term "therapeutically effective dose" is an amount that is sufficient to treat the disease. Amounts of the dose will depend upon the type of cancer to be treated, as well as the stages of the cancer (I-IV).

By "administration at certain intervals" means, for example, that the chimeric monoclonal antibody or antibody-like molecule of the present invention may be administered first, followed by administration of the drugs, radioisotopes, toxins or immunogenic killer cells at an interval later; i.e., 30 minutes, 1 hour, 4 hours, 24 hours later. It also accommodates a situation when the drugs, radioisotopes, toxins or immunogenic killer cells are administered first and then the chimeric monoclonal antibody of the present invention is administered at a time interval later; i.e., 30 minutes, 1 hour, 4 hours, 24 hours later.

The present application discloses novel monoclonal, chimeric or humanized, antibodies, fragments thereof or antibody-like molecules which bind both human acidic and basic ferritin and their use as therapeutic agents either alone, or with drugs, radioisotopes, toxins, immune killer cells or combinations thereof simultaneously or within certain time periods or conjugated to drugs, radioisotopes, toxins, immune killer cells or combinations thereof. The present application further discloses toward nucleic acid sequences which encode the monoclonal, chimeric or humanized, antibodies, fragments thereof or antibody-like molecules and their expression in recombinant hosts.

More specifically the present invention is directed toward monoclonal, chimeric or humanized, antibodies, fragments thereof or antibody-like molecules which specifically bind human acidic and basic ferritin and are derived from parts of the murine AMB8LK antibody.

Murine antibody AMB8LK is a murine antibody that was obtained according to the procedure of Kadouche et al "Analysis of various isoferritins with monoclonal antibodies." C R Seances Acad Sci III; 295 (6) 443-448 (1982). Basically after immunization of female Balb/c mice with ferritin extracted from human spleen, spleen cells of the best responders were fused with murine Sp20 myeloma cells using polyethylene glycol 4000 according to standard protocols and selected hybridomas were cloned, expanded and cultured *in vitro.* AMB8LK was selected for its high affinity of 5. 1 x $10^{-9}$ M, and its specificity for human ferritin.

However, while the murine antibody AMB8LK possesses functional properties which render it suitable as a therapeutic agent, it also is recognized as a foreign antibody in humans and hence is rapidly removed from the circulation. Therefore, higher doses of the murine monoclonal antibody are required for the patient to be treated. This may result in the patient having systemic inflammatory effects.

Therefore, the present invention resolves the problems associated with murine monoclonal antibodies by providing, chimeric or humanized, monoclonal antibodies, fragments thereof or antibody-like molecules having the heavy variable and light variable regions of the murine monoclonal antibody AMB8LK. In a particular embodiment, it is disclosed a chimeric monoclonal antibody comprising the heavy variable and light variable regions of the murine monoclonal antibody AMB8LK, a constant heavy region 1 of human Fab' 2-3 cysteine, a human kappa constant light region and human constant heavy chain regions from human IgG1 or IgG4.

In a first aspect, it is disclosed a polypeptide comprising, from N-terminal to C terminal, SEQ ID NO:30, SEQ ID NO:28 and SEQ ID NO:26 (to form a VH domain) or a polypeptide comprising from N-terminal to C terminal, SEQ ID NO:20, SEQ ID NO:22 and SEQ ID NO:24 (to form a VL domain). In a particular embodiment, applying to both, but independently, VH and VL domains, the different SEQ ID are non-contiguous *i.e*., that each SEQ ID is separated from the other one(s) by additional amino acid residues. These additional amino acid residues can be of murine origin or in the case of humanized antibody design can be derived from human source, especially human immunoglobulin. In this latter case, SEQ ID NOs 30, 28 and 26 and/or SEQ ID Nos: 20, 22 and 24 are grafted on a human scaffold (such as human VH or VL domains) thus replacing their human counterparts. Therefore, the application is directed to VL and VH domains of murine origin as well as VL and VH domains having CDR from murine origin and framework from human origin (also called humanized or CDR grafted).

In a particular aspect, the VH domain has the following formula:

Nter.AAn-SEQ ID NO: 30-AAm-SEQ ID NO:28-AAp-SEQ ID NO:26-AAq.Cter,

wherein "-" represent a peptide bond, AA represents any amino acid, and n equals to 25 or 26, m is an integer comprised between 15 to 19, preferably 17, p is an integer comprised between 35 and 40 and q is an integer comprised between 10 and 12, preferably 11. AAn, AAm, AAp and AAq correspond respectively to FR1, FR2, FR3 and FR4 regions of a heavy variable domain, and can be of murine or human origin. In a particular embodiment, AAn, AAm, AAp and AAq are respectively, and independently from each other, SEQ ID NO:32, SEQ ID NO:34, SEQ ID NO:36 and SEQ ID NO:38.

| | Nucleotide sequence (SEQ ID) | Protein sequence (SEQ ID) |
|---|---|---|
| **AAn** | caggtgcagctgaaggagtcaggacctggcctggtggcac cctcacagagcctgtccatcacatgcactgtctct (31) | QVQLKESGPGLVAPSQSLSITCTVS (32) |
| **AAm** | gtacactgggttcgccagcctccaggaaagggtctggagt ggctgggaacg (33) | VHWVRQPPGKGLEWLGT (34) |
| **AAp** | gactataactcagttctcaaatccagactgagcatcagca aggacaactccaagagccaagttttgttaaaagtgaacag tctacaaactgatgacacagccatatattactgt (35) | DYNSVLKSRLSISKDNSKSQVLLKV NSLQTDDTAIYYC (36) |
| **AAq** | tggggtcaaggaacctcagtcaccgtctcctca (37) | WGQGTSVTVSS (38) |

In a particular embodiment, the VH domain consists essentially of SEQ ID NO:4.

In a particular aspect, the VL domain has the following formula:

Nter.AAr-SEQ ID NO: 20-AAs-SEQ ID NO:22-AAt-SEQ ID NO:24-AAv.Cter,

wherein "-" represent a peptide bond, AA represents any amino acid, and r equals to 25 or 26, s is an integer comprised between 15 to 19, preferably 17, t is an integer comprised between 35 and 40 and v is an integer comprised between 10 and 12, preferably 11. AAr, AAs, AAt and AAv correspond respectively to FR1, FR2, FR3 and FR4 regions of a light variable domain, and can be of murine or human origin. In a particular embodiment, AAr, AAs, AAt and AAv are respectively, and independently from each other, SEQ ID NO:40, SEQ ID **NO:42, SEQ ID NO:44 and SEQ** ID **NO:46:**

| | Nucleotide sequence (SEQ ID) | Protein sequence (SEQ ID) |
|---|---|---|
| **AAr** | caaattgttctcacccagtctccagcaatcctgtctgcat ctctaggggaggagatcaccctaacctgcagtgccagc (39) | QIVLTQSPAILSASLGEEITLT CSAS (40) |
| **AAs** | atgcactggtaccagcagaagtcaggcacttctcccaaac tcttgatttat (41) | MHWYQQKSGTSPKLLIY (42) |
| **AAt** | aacctggcttctggagtcccttctcgcttcagtggcagtg ggtctgggaccttttattctctcacaatcagcagtgtgga ggctgaagatgctgccgattattactgc (43) | NLASGVPSRFSGSGSGTFYSLTISS VEAEDAADYYC (44) |
| **AAv** | ttcggctcggggacaaagttggaaataaaac (45) | FGSGTKLEIK (46) |

In a particular embodiment, the VL domain consists essentially of SEQ ID NO:2.

In a particular embodiment, a polypeptide comprising a VL domain as defined above, such as SEQ ID NO:2 also comprises a kappa or a lambda chain, such as SEQ ID NO:12, to form a light chain. Thus, the C-terminal part of the VL domain is genetically linked to the N-terminal part of SEQ ID NO: 12, possibly via a linker sequence. In a particular aspect, the VL domain of the invention is linked in its N-terminal part to a signal peptide such as the one having SEQ ID NO:14.

In another aspect, a polypeptide comprising a VH domain of the invention, such as SEQ ID NO:4 also comprises a CH1 constant region, such as SEQ ID NO:6. Thus, the C-terminal part of the VH domain is genetically linked to the N-terminal part of SEQ ID NO: 6, possibly via a linker sequence. In a particular embodiment, a sequence comprising a VH domain (such as SEQ ID No:4) and SEQ ID NO:6 also comprises a human Fc region, such as SEQ ID Nos 8 or 10 or a murine Fc region such as SEQ ID No: 18, to form a heavy chain. In that case, the SEQ ID NO:8, 10 or 18 is genetically linked by its N-terminal part to the C-terminal part of SEQ ID NO:6. Independently or in combination with the above embodiments, the VH domain may be linked in its N-terminal part to a signal peptide such as the one having SEQ ID NO:16.

Particular light and heavy chains disclosed in the present applicationare:

- a light chain polypeptide comprising from the N-terminal to the C-terminal, a VL domain of the invention, especially SEQ ID NO:2, and SEQ ID NO:12, and
- a heavy chain polypeptide comprising, from the N-terminal to the C-terminal, a VH domain, such as SEQ ID NO:4, SEQ ID NO:6 and a human Fc region, such as a sequence selected from SEQ ID NO:8, SEQ ID NO:10 and SEQ ID NO:18.

Therefore, the present disclosure relates to a chimeric monoclonal four-chain anti-ferritin antibody comprising two light chains comprising at least a variable light domain (VL) of the invention, of murine origin or having CDR from murine origin and framework from human origin, and particularly the variable light domain as defined in SEQ ID NO: 2 or a variant thereof, and two heavy chains comprising at least a variable heavy domain (VH) of the invention, of murine origin or having CDR from murine origin and framework from human origin, and particularly the variable heavy domain as defined in SEQ ID NO: 4 or variant thereof. The combination of these VH and VL domains forms the ferritin binding site. In a particular embodiment, the variable light domain of the chimeric anti-ferritin monoclonal antibody is the one as defined in SEQ ID NO:2 and/or the variable heavy domain of the chimeric anti-ferritin monoclonal antibody is the one as defined in SEQ ID NO:4.

In a particular aspect of the disclosure, a chimeric anti-ferritin monoclonal antibody comprises, besides a variable light and heavy domains of the invention (such as SEQ ID Nos 2 and 4 respectively), at least a constant human CH1 region, such as a sequence of SEQ ID NO:6 and/or a lambda or kappa region, such as SEQ ID NO:12. Therefore, SEQ ID NO:6 is genetically linked to the variable heavy domain (such as SEQ ID NO:4) and/or SEQ ID NO:12 is genetically linked to the variable light domain (e.g. SEQ ID NO:2).

Besides the light and heavy domains derived from the variable region of AMB8LK, the chimeric monoclonal antibody of the present application may also includes the constant heavy region 1 of Fab'2-3 cyst of SEQ ID NO: 6, as well as the human kappa light constant region of SEQ ID NO: 12 and a heavy constant Fc region, such as the human gamma 1 constant region (SEQ ID No: 8) or the human gamma 4 constant region (SEQ ID NO: 10) or the murine $\gamma$2a constant region (SEQ ID NO: 18).

The application also relates to a humanized monoclonal antibody in which the light chains are a hybrid of murine CDR and human FR, and/or the heavy chains are a hybrid of murine CDR and human FR. In another aspect, the humanized monoclonal antibody consists of:

- two human light chains in which the CDR1, CDR2 and CDR3 have been respectively replaced by SEQ ID NO:20, SEQ ID NO:22 and SEQ ID NO:24, and/or
- two human heavy chains in which the CDR1, CDR2 and CDR3 have been respectively replaced by SEQ ID NO:30, SEQ ID NO:28 and SEQ ID NO:26.

Briefly, a human immunoglobulin sequence is isolated from databases for its high sequence similarity with the CDR and/or FR domains of the murine antibody, especially the CDR and/or FR domains as defined by their SEQ ID in the present application. At least one, preferably all, CDR of the VL domain and/or the VH domain of the human immunoglobulin sequence is(are) replaced by the corresponding murine CDR, either by genetic engineering or by chemical synthesis of the full-length sequence. Optionally, tridimensional *in silico* analysis can be carried out to modify (by substitution, addition and/or deletion) one or more amino acids of the human framework by the corresponding amino acid(s) found at the same position(s) in the murine framework (independently in the VL and VH domain).

Also encompassed, in the invention, are functional fragments of this four-chain, chimeric and humanized, monoclonal antibody, provided that these fragments still bind the ferritin epitope, as defined above. These fragments include Fv fragment (non-covalent association of the VH and VL domains of the invention) and Fab fragment, as defined above.

The invention is also directed to molecules based upon Fab fragment as defined above, such as bispecific or trispecific antibodies (see definition above), or bifunctional or trifunctional antibodies. In a particular embodiment, a Fab as defined above is linked to a ligand such a cytokine, a receptor or any protein of interest, to form a bifunctional antibody.

The invention also relates to antibody-like molecules as defined above and comprising at least one variable light domain (VL) of the invention, murine or human/murine hybrid, and particularly the variable light domain as defined in SEQ ID NO: 2 or a variant thereof in combination with at least one variable heavy domain (VH) of the invention, murine or human/murine hybrid, and particularly the variable heavy domain as defined in SEQ ID NO: 4.

In a particular embodiment, the C-terminal part of the variable light domain (VL) of the invention is linked (directly or via a linker) to the N-terminal part of the variable heavy domain (VH) of the invention, to form a scFv. In another embodiment of scFv, the C-terminal part of the variable heavy domain (VH) of the invention is linked (directly or via a linker) to the N-terminal part of the variable light domain (VL) of the invention. These scFv can then be used to form Bis-scFv or diabody, with other scFv recognizing the same or a different epitope and/or antigen. All other molecules based on these scFv can also be envisaged by the person skilled in the art, as long as said antibody-like molecule retains the ferritin binding activity.

In another aspect, the present application is directed to a chimeric anti-ferritin monoclonal antibody comprising:

- two heavy chains comprising a VH domain sequence encoded by a polynucleotide comprising from 5' to 3' SEQ ID NO:29, SEQ ID NO:27 and SEQ ID NO:25, and
- two light chains comprising a VL domain sequence encoded by a polynucleotide comprising from 5' to 3', SEQ ID NO:19, SEQ ID NO:21 and SEQ ID NO:23.

In another aspect, the VL domain polynucleotide consists essentially of (SEQ ID NO:1 and/or the VH domain polynucleotide consists essentially of SEQ ID NO:3.

A particular chimeric anti-ferritin monoclonal antibody, for example, comprises:

- two heavy chains encoded by a polynucleotide comprising from 5' to 3' SEQ ID NO:29, SEQ ID NO:27 and SEQ ID NO:25 linked to a polynucleotide encoding for a CH1 constant region such as SEQ ID NO:5 linked to a polynucleotide encoding a human Fc region such as the one selected from the group consisting of SEQ ID NO:7 and SEQ ID NO:9 , and
- two light chains encoded by a polynucleotide comprising from 5' to 3' SEQ ID NO:19, SEQ ID NO:21 and SEQ ID NO:23 linked to a polynucleotide encoding for a lambda or kappa region such as SEQ ID NO:11.

In yet another aspect, the heavy chain is encoded by a polynucleotide comprising, from 5' to 3', SEQ ID NO:3 linked to SEQ ID NO:5 linked to a polynucleotide encoding a human Fc region such as the one selected from the group consisting of SEQ ID NO:7 and SEQ ID NO:9. In another aspect, the light chain is encoded by a polynucleotide comprising from 5' to 3' SEQ ID NO:1 linked to SEQ ID NO:11.

In yet another aspect, the monoclonal, chimeric or humanized, anti-ferritin antibody or fragments thereof as well as the antibody-like molecules of the present invention are coupled to a bioactive agent such as a drugs, radioactive isotopes, toxins, or immune killer cells such as natural killer cells or natural killer T cells. This coupling may be through linkers such as amino acids, β-glucuronide linkers such as those described in Jeffrey et al "Development and Properties of β-Glucuronide linkers for Monoclonal Antibody-Drug conjugates, Bioconjugate Chem. 17 (3) 831-840 (2006) and U.S. Patent 7,098,308.

Drugs that can be either administered with the monoclonal antibody or the antibody-like molecule of the present invention or conjugated to this monoclonal antibody or the antibody-like molecules include alkylating agents such as nitrogen mustards, chlorambucil, chloromethane, cyclophosphamide isofamide, melphalen and the like; nitrosouces such as carmustine, fotemustine, lomustine and the like; platinum drugs such as cisplatinum, oxaliplatin, BBR3464, Busulfan, ThioTepa and the like; antimetabolites such as folic acid drugs of aminopterin, methotrexate and the like; purine drugs such as cladribine, clofarabine and the like; and pyrimidine drugs such as fluorouracil, capecitabine, cytarabine, floxuridine, gemcitabine and the like. Mixtures of these drugs can also be used with the monoclonal antibody or the antibody-like molecule of the present disclosure, depending on the type of cancer to be treated. For instance, gemcitabine and paclitaxel can be administered to patients having breast cancer with the monoclonal antibody or the antibody-like molecule of the present disclosure.

Radioactive isotopes that can be conjugated to monoclonal antibodies or antibody-like molecules include gamma, beta, alpha, alpha-beta and beta-gamma emitters as defined in the following Table:

| Radioisotope | Emitted radiation | Note |
|---|---|---|
| $^{211}At$ | Alpha | |
| $^{213}Bi$ | Alpha-Beta-Gamma | Use for alpha radiation |
| $^{51}C$ | Gamma | |
| $^{64}Cu$ | Beta-Gamma | |
| $^{165}Dy$ | Beta | |
| $^{169}Er$ | Beta | |
| $^{18}F$ | Beta-Gamma | |
| $^{68}Ga$ | Beta-Gamma | |
| $^{67}Ga$ | Beta-Gamma | Use for gamma radiation |
| $^{166}Ho$ | Beta-Gamma | Use for both types of radiations |
| $^{111}In$ | Gamma | |
| $^{123}I$ | Gamma | |
| $^{125}I$ | Beta-Gamma | Use for both types of radiations |
| $^{131}I$ | Beta-Gamma | Use for both types of radiations |

(continued)

| Radioisotope | Emitted radiation | Note |
|---|---|---|
| $^{192}Ir$ | Beta-Gamma | |
| $^{59}Fe$ | Beta-Gamma | |
| $^{177}Lu$ | Beta-Gamma | |
| $^{32}P$ | Beta | |
| $^{42}K$ | Beta-Gamma | |
| $^{186}Re$ | Beta-Gamma | |
| $^{188}Re$ | Beta-Gamma | Use for beta radiation |
| $^{153}Sm$ | Beta-Gamma | Use for beta radiation |
| $^{75}Se$ | Gamma | |
| $^{24}Na$ | Beta-Gamma | |
| $^{89}Sr$ | Beta | |
| $^{99m}Tc$ | Gamma | |
| $^{201}Ti$ | Beta-Gamma | |
| $^{133}Xe$ | Beta | |
| $^{90}Y$ | Beta | |

These isotopes can be linked to the monoclonal, chimeric or humanized, anti-ferritin antibody or fragments thereof as well as the antibody-like molecules of the disclosure through chelating agents such as bifunctional ligands, diethylenetriaminepentaacetic acid (DTPA) and any of its derivatives, 1,4,7,10-tetraazacyclododecane-1,4,7,10-tetraacetic acid (DOTA) and any of its derivatives, or any macrocyclic ligand, 1,3 bis[N-[N-(2-aminoethyl)-2-aminoethyl]-2-aminoacetamido]-propane-N,N,N',N",N"',N"",N""',N""'-octaacetic acid (LiLo),N-(S-acetylmercaptoacetyl)(p-NCS) phenylalanylglycylglycine ethyl ester (for $^{99m}$TC or $^{186}$Re labeling), 5,7-dioxo-1,11-carboxymethyl)-1,4,8,11-tetraazacyclotridecane, 1,4,7,10-tetraazacyclotridecane-N,N',N",N"'-tetraacetic acid (TRITA) 1, 4, 8, 11-tetraaxacyclotetra-decane-N, N', N", N"'-tetraacetic acid (TETA), 1,5,9,13-tetraazacyclohexa decane-N,N',N"",N"""-tetraacetic acid (HETA).

Examples of toxins include Coley toxins, ricin A chain, PAP (pokeweed antiviral toxin), *Staphylococcus aureus* bacteria (SEB), *Pseudomonas exotoxin,* and the like. The toxins can be coupled to the monoclonal antibody of the present disclosure or they can be given simultaneously therewith.

Besides drugs and radioisotopes, other biological molecules can also be coupled to the monoclonal, chimeric or humanized, anti-ferritin antibody or fragments thereof as well as the antibody-like molecules of the present disclosure such as enzymes, nucleic acids, anti-sense RNAs, siRNA, biotin, streptavidin or avidin molecules to improve antibody binding specificity, or biological molecules that can bind to a specific cell type and the like.

It is noteworthy that at least two monoclonal, chimeric or humanized, antibodies, fragments or antibody-like molecules of the disclosure can be combined in any combination in a single composition.

In another aspect the present disclosure relates to a composition comprising (a) a chimeric anti-ferritin monoclonal antibody, a humanized monoclonal antibody, a functional fragment, a bispecific monoclonal antibody, a trispecific monoclonal antibody, a bifunctional antibody, a scFv molecule, a Bis-scFv or a diabody of the invention, such as a monoclonal antibody comprising as a light chain SEQ ID Nos. 2 and 12, and as a heavy chain SEQ ID Nos 4, 6 and 8 or SEQ ID Nos. 4, 6 and 10, and (b) a pharmaceutically acceptable vehicle.

Pharmaceutically acceptable vehicles include carriers, excipients and stabilizers. The formulations can be prepared as set forth in Remington's Pharmaceutical Sciences 16th edition, Osol A. editor (1980). Examples of carriers, excipients and stabilizers include saline, PBS, buffers such as phosphate, citrate and other organic acids; antioxidants such as ascorbic acid, low molecular weight polypeptides; proteins such as serum albumin, immunoglobulins, gelatins; hydrophilic polymers such as PVP; amino acids of glycine, glutamine, arginine, lysine or asparagines; carbohydrates such as glucose, mannose or dextrins; sugar alcohols including mannitol or sorbitol; salt-forming counterions such as sodium and/or nonionic surfactants such as Tween®.

The pharmaceutical composition can be administered by infusion, intravenously, intraperitonally, intramuscularly intraarterially or by sustained release systems such as liposomes or polylactic-coglycolic acid. Sustained release systems are described in U.S. Patent 5,255,212 and Lewis, "Controlled release of bioactive agents from lactide/glycolide polymer." In M. Chasin and R. Longer (Eds.) Biodegradble Polymers as Drug Delivery Sysytems (Marcel Decker: New York, 1990).

Dosages and drug concentrations can be determined depending on the particular use envisioned, the stage and type of cancer to be treated, as well as the body weight of the mammal. Standard pharmaceutical procedures can be used to determine the toxicity and therapeutic efficacy by using cell cultures or experimental animals. Usually the $LD_{50}$ (lethal dose to 50% of the population) and the $ED_{50}$ (the therapeutically effective dose in 50% of the population) can be determined. The therapeutic index is the dose between the toxic and therapeutic effects.

The data obtained from the above assays can be used in a range of dosage for human and mammalian use. Generally from 1 ng/kg to 100 mg/kg doses are administered.

The present application, also discloses a method of delivering a drug, radioactive materials, toxins, immune killer cells or combinations thereof to cells containing basic and acidic ferritin, said method comprising administering to a mammal in need of such treatment a composition comprising (a) a chimeric anti-ferritin monoclonal antibody, a humanized monoclonal antibody, a functional fragment, a bispecific monoclonal antibody, a trispecific monoclonal antibody, a bifunctional antibody, a scFv molecule, a Bis-scFv or a diabody as disclosed herein, in particular an antibody comprising as a light chain SEQ ID Nos. 2 and 12, and as a heavy chain SEQ ID Nos 4, 6 and 8 or SEQ ID Nos. 4, 6 and 10 and (b) a composition selected from the group of drugs, radioactive isotopes, toxins, immune killer cells and combinations thereof, wherein said composition is in a pharmaceutically acceptable carrier.

The chimeric construct having SEQ ID NO: 8 has ADCC effector function, to enhance the performance of this monoclonal antibody, a radioactive isotope or an antimetabolite drug such as gemcitabine can also be used in the formulation.

As discussed above the drug, radioactive isotopes, toxins immune killer cells or combinations thereof may be attached to the monoclonal antibody, the fragment or the antibody-like molecule as disclosed herein, either through a chelating agent or a linker or they can be administered simultaneously or at specific intervals with the monoclonal antibody, the fragment or the antibody-like molecule of the present disclosure.

In yet another aspect, the present application discloses a method of treating a cancer selected from the group of pancreatic cancer, Hodgkin's lymphoma, Kaposi's sarcoma and hepatocellular carcinoma said method comprising administering to a mammal in need of such treatment (a) a chimeric anti-ferritin monoclonal antibody, a humanized monoclonal antibody, a functional fragment, a bispecific monoclonal antibody, a trispecific monoclonal antibody, a bifunctional antibody, a scFv molecule, a Bis-scFv or a diabody as disclosed herein, in particular an antibody comprising as a light chain SEQ ID Nos. 2 and 12, and as a heavy chain SEQ ID Nos 4, 6 and 8 or SEQ ID Nos. 4, 6 and 10, and (b) a pharmaceutically acceptable carrier and optionally a composition selected from the group of drugs, radioactive isotopes, toxins, immune killer cells and combinations thereof.

In another aspect, the present application relates to nucleic acids (or polynucleotides) encoding any variable light domain (VL), any variable heavy domain (VH), any light chain, any heavy chain or any polypeptide as described in the present application.

In another aspect, a nucleic acid encoding a variable light domain comprises, from 5' to 3', SEQ ID NO: 19, SEQ ID NO:21 and SEQ ID NO:23. In yet another aspect, these SEQ ID are non contiguous. In another aspect, the nucleic acid consists essentially of or comprises SEQ ID NO:1 or any polynucleotide variant thereof.

In another aspect, a nucleic acid encoding a variable heavy domain comprises, from 5' to 3', SEQ ID NO: 29, SEQ ID NO:27 and SEQ ID NO:25. In yet another aspect, these SEQ ID are non contiguous. In another aspect the nucleic acid consists essentially of or comprises SEQ ID NO:3 or any polynucleotide variant thereof.

In another aspect, a polynucleotide encoding a variable light domain of the invention (such as SEQ ID NO:1) also comprises a kappa or lambda chain sequence, such as SEQ ID NO:11. Thus, the 3' part of the variable light domain polynucleotide is genetically linked to the 5' part of SEQ ID NO: 11, possibly via a linker sequence. In another aspect, the variable light domain polynucleotide is linked in its 5' part to a signal peptide such as the one having SEQ ID NO:13.

In yet another aspect of the present disclosure, a polynucleotide encoding a variable heavy domain of the present application (such as SEQ ID NO:3) also comprises a human CH1 constant region sequence, such as SEQ ID NO:5; Thus, the 3' part of a variable heavy domain polynucleotide is genetically linked to the 5' part of SEQ ID NO: 5, possibly via a linker sequence. In yet another aspect, a polynucleotide comprising a polynucleotide encoding a variable heavy domain of the present application (such as SEQ ID NO:3) and SEQ ID NO:5 also comprises a sequence encoding a human Fc region, such as SEQ ID Nos 7, 9 or a murine Fc region (SEQ ID No:17). In that case, the SEQ ID NO: 7, 9 or 17 is genetically linked by its 5' part to the 3' part of SEQ ID NO:5. Independently or in combination with the above embodiments, a variable heavy domain polynucleotide is linked in its 5' part to a signal peptide such as the one having SEQ ID NO:15.

Particular nucleic acids are:

- a polynucleotide comprising from 5' to 3' a variable light domain polynucleotide disclosed herein (such as SEQ ID NO:1) and SEQ ID NO:11,

- a sequence comprising from 5' to 3' SEQ ID NO:13, a variable heavy domain polynucleotide disclosed herein and SEQ ID NO:11,
- a sequence comprising from 5' to 3' a variable heavy domain polynucleotide disclosed herein (such as SEQ ID NO:3), SEQ ID NO:5 and a polynucleotide encoding a Fc region, such as a sequence selected among SEQ ID NO:7, SEQ ID NO:9 and SEQ ID NO:17, and
- a sequence comprising from 5' to 3' SEQ ID NO:15, a variable heavy domain polynucleotide of the invention, SEQ ID NO:5 and a polynucleotide encoding a Fc region, such as a sequence selected among SEQ ID NO:7, SEQ ID NO:9 and SEQ ID NO:17.

Another sequence is the one encoding the ScFv disclosed above and having a variable light domain polynucleotide of the invention (such as SEQ ID NO:1) genetically linked to a variable heavy domain polynucleotide disclosed herein (such as SEQ ID NO:3), provided the scFv retains the ferritin-binding activity.

Isolated nucleic acids having at least 95% or 98% or 99% sequence identity to variable heavy and light domain polynucleotides disclosed herein, and to SEQ ID NOS. 1 and 3, are also encompassed in an aspect of the present disclosure. In the nucleic acids or polynucleotides defined above, the various SEQ ID may be replaced, individually and independently one from the other, by a variant having at least 85%, 90%, 95% or 99% sequence identity with SEQ ID NO: 1, 3, 5, 7, 9, 11, 13, 15, 17, 19, 21, 23, 25, 27, 29, 31, 33, 35, 37, 39, 41, 43 and 45, with the proviso that said various domains encoded by these nucleotide sequences enables the resulting monoclonal antibody or antibody-like molecules to keep the AMB8LK binding activity to human acidic and basic ferritin. Polynucleotide variants are variants containing conservative substitutions i.e., a nucleotide substitution that do not modify the nature of the encoded amino acid. Other particular nucleotide substitution are semi-conservative *i.e.,* that the encoded amino acid is from the same class as the original amino acid, as detailed above.

The application further relates to nucleic acids encoding the polypeptide or which hybridize, optionally over the full-length sequence, to a DNA sequence consisting of the nucleotide sequence encoding such polypeptide under stringent conditions. These stringent conditions are described by Sambrook et al, Molecular Cloning Manual, 3$^{rd}$ edition (2001), i.e., as an example, the following conditions: hybridization buffers: 2 X SSC, 10 X Denhardts solution (Ficoll 400 & PEG & BSA, ratio 1:1:1), 0.1% SDS, 5 mM EDTA, 50 mM $Na_2HPO_4$, 250 $\mu$g/ml herring sperm DNA, 50 $\mu$g/ml of t-RNA or 0.25 M of sodium phosphate buffer with a pH of 7.2, 1 mM EDTA, 7% SDS;

Hybridization temperature: 60°C;
Washing buffer: 2 X SSC, 0.1% SDS;
Washing temperature: 60°C.

The application further relates to recombinant nucleic acid vectors comprising at least one nucleic acid sequence as defined in the present application, and in vectors comprising a variable heavy domain polynucleotide disclosed herein and/or a variable light domain polynucleotide disclosed herein, and vectors comprising SEQ ID NO:1 and/or SEQ ID NO:3.

For example a vector comprising a nucleic acid encoding a light and a heavy chains as disclosed herein comprises the heavy variable and light variable domains of the murine monoclonal antibody AMB8LK, a constant heavy region 1 of human Fab' 2-3 cysteine, a human kappa constant light region and an Fc region constant heavy chain region from human IgG1 or IgG4 or a murine $\gamma$2a.

The vectors can be cloning vectors such as plasmids or modified viruses, but can also be bacteriophages such as lambda derivatives, pBR322, pUC plasmid derivatives of the Bluescript vector. In another embodiment, the nucleic acids are inserted into baculovirus plasmid vectors such as pVL941.

In yet another embodiment, the vector comprises a nucleic acid encoding the light chain described herein. In another aspect, the vector comprises a nucleic acid encoding the heavy chain described herein. In another embodiment, the vector comprises a nucleic acid encoding the heavy chain and the light chain described herein.

Methods of producing antibodies or antibody-like molecules of the present disclosure comprising growing cells containing the above recombinant vectors is also an aspect of the present application. In this regard, the cells can be CHO cells, *E. coli,* yeast cells, VERO cells, HELA cells, *COS* cells, CR cells: 1650, W138, BHK, HepG2, 3T3 , A549, PC12, K562, 293 cells, insect cells such as *Spodotera frugiperda* Sf9 cells (ATCC 358 CRL 1711), Cv1 cells and the like. According to the nucleic acid inserted in the vectors, and the number of vectors transduced into the cells, the protein or the antibody or the antibody-like molecule is expressed in the cell and can then be recovered by methods known in the art such as via columns.

Any expression regulatory sequences, such as promoters can be used in the vectors of the present disclosure. Examples of such promoters include SV40 early promoters, the promoter contained in the 3'long terminal repeat of Rous sarcoma virus, the herpes thymidine promoter, the regulatory sequence of the mettallothionein gene, prokaryotic expression vectors such as the beta-lactamase promoter or the lac promoter and the like, as well as polyhedrin promoter (Ph) or promoter10 (both adapted for baculovirus expression).

The chimeric monoclonal antibodies, fragments or antibody-like molecules of the present disclosure can be synthesized using chemical methods based on the sequences described herein. These methods are known in the art and are described, for example in Hunkapiller et al., Nature 310:105-111.

The chimeric monoclonal antibodies, fragments or antibody-like molecules of the present disclosure can be obtained by recombinant techniques. Therefore, the application also relates to monoclonal antibodies, fragments or antibody-like molecules as defined above, obtained by expression of at least one nucleic acid of the invention. In this case, construction of suitable vectors containing the desired coding and control sequences employ standard ligation techniques. Isolated plasmids or DNA fragments are cleaved tailored and relegated in the form desired to form the plasmid required.

Cleavage of the sequences is completed using appropriate endonucleases (restriction enzymes) in a suitable buffer. These cleavage techniques are well known to those skilled in the art. If blunt ends are required the preparation is treated at 15°C for 15 minutes with 10 units of E. coli DNA Polymerase (Klenow fragment), phenol-chloroform extracted and ethanol precipitated.

Size separation of the fragments can be carried out as described by Goeddel, D. et al Nucleic Acid Res. 8:4057 (1980). For ligation approximately equimolar amounts of the desired components suitably end tailored to provide correct matching are treated with 10 units T4 DNA ligase per 0.5 $\mu$g DNA.

The expression vectors constructed are then used to transform suitable cells. The light and heavy chains are transformed in a same cell, either two vectors each bearing the light or the heavy chain or a single vector containing both genes and able to express both the light and heavy chains.

The cells are then grown under conditions for production of the desired protein. The protein is then recovered from the cell culture by methods known in the art. The recovery process depends on the type of cells used for protein production. When light and heavy chains are coexpressed the isolation procedure is planned to recover the constituted antibody. When signal peptides are present in the N-terminal part of the heavy and light variable domains, they are cleaved into the cells, before assembly of the light and heavy variable domains and if appropriate chains comprising them.

For constructing the monoclonal antibodies, fragments or antibody-like molecules, the desired portions of the genes encoding the light and heavy chains (or variable light and heavy domains) from suitable sources are religated using ligases. Thus, the sources of the heavy chain gene and the light chain gene (or VH and VL) which encode the variable portions produced by the murine hybridoma AMB8LK are recovered and cloned. From this culture and gene fragments encoding the constant regions of the heavy and light chains of human gamma 1, human gamma 4, human kappa and Fab'2 are recovered and cloned from human myeloma cells. Suitable restriction enzymes are used to ligate the variable portions to the light portions of the mouse gene to the constant portions of the human gene for each of the two chains.

Once the monoclonal antibodies, fragments or antibody-like molecules of the present disclosure are fabricated as described above with or without drugs, radioisotopes, toxins, natural killer cells and combinations thereof, they can be tested for immunoreactivity with human ferritin using ELISA or RIA assays as set forth in the Examples. This is especially helpful when variants of the sequences are used and/or fragments.

A number of embodiments of the invention have been described, as shown below.

**Example 1- Monoclonal anti-ferritin antibody AMB8LK**

AMB8LK is a monocolonal IgG1 anti-ferritin antibody at 1-10 mg/ml in PBS. This antibody was obtained as previously described by Kadouche et al "Analysis of various isoferritins with monoclonal antibodies]. C R Seances Acad Sci III 1982;295 (6):443-8, after immunization of female Balb/c mice with ferritin extracted from human spleen. Spleen cells of the best responders were fused with murine Sp2/0 (ATCC Number: CRL-1581) myeloma cells using polyethylene glycol 4000 according to standard protocols and selected hybridomas were cloned, expanded and cultured *in vitro.* AMB8LK was selected for its very high affinity of 5.1 x $10^{-9}$ M, and its specificity for human ferritin.

**Example 2- Conjugation of the DPTA and DOT A chelates on AMB8LK**

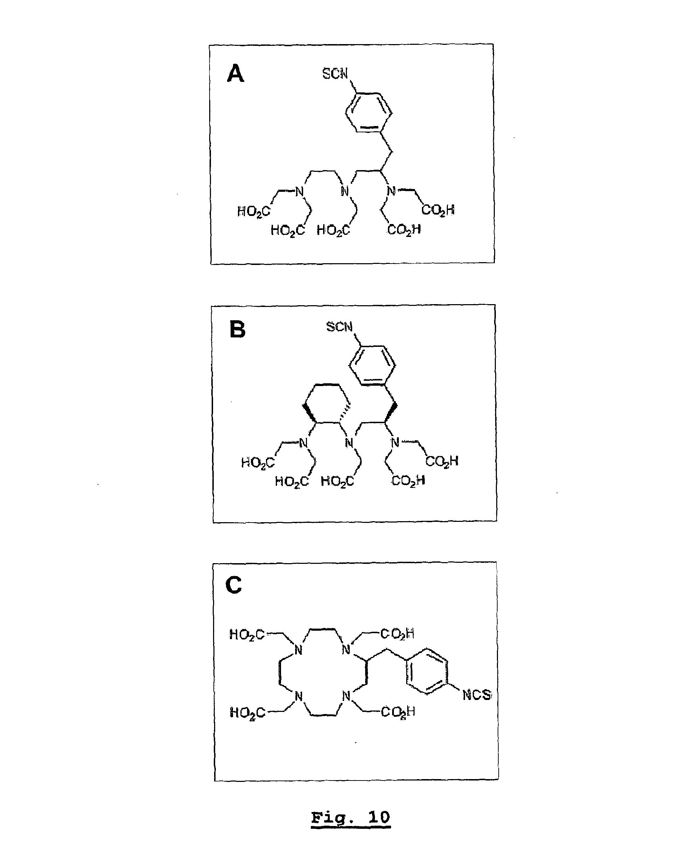

The bifunctional chelators *p*SCN-Bz-DTPA ( 2-(4-isothiocyanatobenzyl)-diethylenetriaminepentaacetic acid), *p*SCN-Bz-CHX-A"-DTPA ((R)- 2-amino-3-(4-isothiocyanantophenyl)propyl]-trans-(S,S)-cyclohexane-1,2-diamine-pentaacetic acid) and *p*SCN-Bz-DOTA (2-(4-isothiocyanatobenzyl)-1,4,7,10-tetraazacyclododecane-1,4,7,10-tetraacetic acid) (Fig. 10) were purchased from Macrocyclics. These ligands were then conjugated to AMB8LK as previously described by Cooper et al.,. "Conjugation of chelating agents to proteins and radiolabeling with trivalent metallic isotopes." Nature Protocols 2006; 1:314-17.. Briefly, the antibody was preincubated at room temperature with EDTA, transferred to HEPES 0.1 M, pH8.5 and the concentration was adjusted to 5-10 mg/ml. Solutions of the chelators in ethanol were added drop wise to the antibody solution at 50 equivalents of chelate/antibody, unless stated otherwise. The reaction was allowed to proceed overnight at 37°C. The buffer was then changed into ammonium acetate buffer 0.1M, pH 6 and removal of the unbound chelator was performed using ultrafiltration with a molecular weight cutoff of 30 kDa. The final concentration of the antibody was determined by UV absorbance at 280 nm. For ease of description Bz-DTPA- AMB8LK is described below as DTPA-AMB8LK; the Bz-CHX-A-DTPA-AMB8LK as CHX-DPTA"-AMB8LK and the Bz-DOTA-AMB8LK is designated DOTA-AMB8LK.

The number of chelates conjugated to the antibody was determined using a $^{57}$Co assay as described (Meares et al.,"Conjugation of antibodies with bifunctional chelating agents: isothiocyanate andbromoacetamide reagents, methods of analysis, and subsequent addition of metal ions." Anal Biochem 1984; 142 (1):68-78). A constant amount of $^{57}CoCl_2$ of known specific activity (ICN, Basingstoke, Hamps, London) was incubated with increasing amounts of conjugated-antibody for 1-2 hours at room temperature or 37 °C for the DOTA-conjugate. The reaction was stopped by adding 50 mM EDTA in 0.1 ammonium acetate, 2-10% vol/vol. The solutions were analyzed by instant thin-layer chromatography (ITLC) (Pall Life Sciences, Portsmouth, U.K.) using the EDTA solution as the mobile phase. Unbound Co migrated to the solvent front, whereas $^{57}$Co-labeled antibody remained at the origin of the strip. The activity in each portion of the strip was measured with a 1282 gamma counter (LKB, Wallac). Linear regression of the results using the equation % of bound activity - f(Ab amount) allowed the determination of the mean number of chelates conjugated to each molecule of antibody.

Optimal conditions for chelator conjugation were determined after incubating equal amounts of AMB8LK with increasing amounts of pSCN-Bz-DTPA at 4°C, RT or 37°C for various times. To achieve a conjugation of *circa 3* chelates substitutions per antibody, a chelate per antibody reaction ratio of 50:1 was used, incubated overnight at 37°C. Overnight incubation at room temperature produced a yield of about 1.7 DTPA/antibody (Ab) and incubation at 37°C for 6 hours, about 1.5 DTPA/Ab. The efficiency of conjugation was similar for the three chelators: effective conjugation of 3 Bz-DTPA per Ab, 3 Bz-CHX-A'-DTPA per Ab and 3.2 Bz-DOTA per Ab were achieved. Less than 4% antibody aggregates were formed during the conjugation process as determined by SE-HPLC.

**Example 3- Size-Exclusion Chromatography**

Size-exclusion high liquid performance chromatography (SE-HPLC) analysis was performed using a Beckman 114M solvent module-pump with a Beckmamn 340 injection module and a Beckman 160 absorbance detector connected to a Raytest gamma radioactivity detector. Stationary phase was a BioSep SEC-S3000 column 300 x 7.8 mm (Phenomex, Cheshire, UK). Isocratic elution with 0.1 M phosphate buffer (0.06 M $Na_2HPO_4$, 0.04 M $NaH_2PO_4$, 2 mM EDTA, pH 7) was used at a flow rate of 0.5 ml/min. Chromatograms were analyzed using Galaxie (Novell) software.

Example 4- Radiolabelling with indium and yttrium

Indium [$^{111}$In] chloride (gamma emitter), $^{111}$MBq in 500 $\mu$l 0.05 HCl was purchased from Mallinckrodt (Petten, Netherlands). Yttrium [$^{90}$Y] chloride (pure beta emitter) 185MBq, was acquired from MDS Nordion (Fleurs, Belgium) and dissolved in HCL 0.05 M to give a final volume of 100 $\mu$l.

The pH of indium or yttrium was adjusted to 6 by adding 0.1 M ammonium acetate, pH 6 (20% of the final volume). The conjugated antibodies were added to obtain a specific activity of 130 MBq/mg antibody (unless stated otherwise) and incubated for 30-60 minutes at room temperature for the DTPA-conjugates or at 37°C for the DOTA. The labeling reactions typically were performed using 50 $\mu$g of antibody with 6.5 MBq of radionuclide. The reaction was then quenched by adding 2-10% vol/vol EDTA 50mM and PBS was added to produce a concentration of about 10 MBq/ml. Labelling efficiency was determined by TLC using 50 mM EDTA in 0.1 M ammonium acetate, pH 6 as the mobile phase. Under these conditions radiolabelled antibodies have an $R_f$ =0 while unbound radionuclide has an $R_f$ =1. The percentage activity was measured after cutting the strip in two and counting the activity in each portion in a 1282 gamma counter (LKB, Wallac, Finland). Labelling efficiency was expressed as (cpm origin)/cpm origin + cpm front) X 100. Labelling efficiency was also determined by HPLC using an isocratic mobile phase with phosphate buffer, then integrating the peaks with the Galaxie software (Novell).

Increasing the incubation time to 1 hour did not improve significantly the labelling efficiency while increasing the temperature to 37°C for the DTPA-conjugates improved the labelling efficiency by only 1% compared to labelling at room temperature. After having assessed the time and temperature parameters, specific activities ranging from 40-330 MBq/mg of Ab were tested. No significant difference in the labelling results was observed. The effect of radioactive concentration during the labelling reaction was also studied. The results of indium labelling using radioactivity concentrations ranging from 100 to 330 MBq/ml demonstrated that within this concentration range, labelling efficiencies were similar.

**Example 5- *In vitro* stability of the radiolabelled immunoconjugates**

Stability of the $^{111}$In and $^{90}$Y-labelled immunoconjugates was assessed in PBS and in plasma, at 4°C and 37°C, respectively. Radiolabelling was performed as described above, then 4-6 MBq of the radiolabelled-monoclonal antibody was added to PBS or plasma to produce a final volume of 0.4-0.5 ml. 10 $\mu$l (0.05 MBq) of the radiolabelled antibody was analyzed by SE-HPLC and 0.5 ml eluate fractions were collected for 30 minutes. The activity in each fraction was counted using a gamma counter and the elution profile was plotted from these results. The percentage of radiolabelled antibody remaining over time was then determined, For the stability study in PBS, in addition to SE-HPLC, ITLC was performed using 50 mM EDTA as the mobile phase.

The two $^{111}$In-DTPA-immunoconjugates stored at 4°C in PBS showed greater than 98% stability over 7 days, while the $^{111}$In-DOTA conjugate showed 94%. The stability of the $^{90}$Y-labelled-antibodies stored in the same conditions was around 80%. In plasma at 37°C, the indium- and yttrium-compounds were stable for at least 7 days (loss of less than 3% of activity).

**Example 6- Immunoreactivity of these immunoconjugates on pure ferritin: ELISA and RIA**

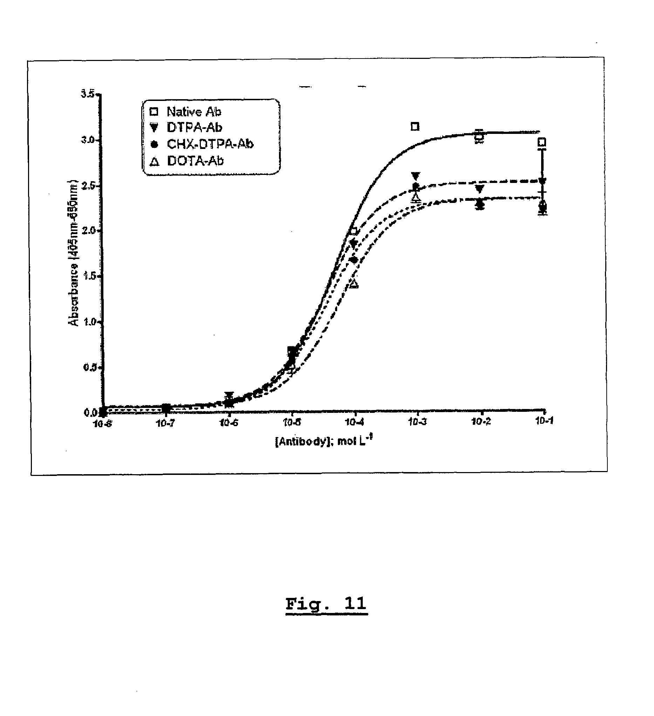

The immunoreactivity of the three immunoconjugates was assessed using an ELISA. Preliminary assays using ferritin (human liver ferritin, Calbiochem) adsorbed overnight at 0.1, 1.5 and 10 $\mu$g/ml showed that the optimal ferritin concentration for the assay was 5 $\mu$g/ml ferritin. Thus, 5 $\mu$g/ml ferritin in PBS was adsorbed on a 96-well plate (Maxisorp, Nunc). The wells were washed with PBS-0.4% Tween-20 then blocked for 2 hours at room temperature with PBS-0.5% BSA, Conjugated antibodies and the unconjugated antibody used as a control were incubated for 2 hours at room temperature in PBS-0.5% BSA, at concentrations ranging from $10^{-8}$ to $10^{-1}$ mg/ml (50 $\mu$l per well). Wells were then washed with PBS-0.4% Tween-20 and the secondary antibody (alkaline phosphatase conjugated IgG anti-mouse, Sigma) was added at 1:25,000 dilution in PBS for 1 hour at room temperature. After washings the substrate p-nitrophenyl phosphate tablets, pNPP, Sigma) was added (1 mg/ml pNPP in 0.2 M Tris buffer, 5 mM magnesium chloride). The reaction was quenched after 30 minutes with 3 M NaOH, 1/1 vol/vol. Absorbance was read at 405 nm on a DTX 880 plate reader (Beckman Coulter) and results were analyzed using the GraphPad Prism software (San Diego, California). Results showed a slight decrease in the binding of ferritin of the conjugates compared to that of native antibody (Fig. 11). However, no difference could be noticed between the three compounds.

A solid-phase radioimmunoassay was performed using $^{111}$In- and $^{90}$Y-labelled antibodies. Maxisorp tunes (Nunc) were incubated overnight at 4°C with 1.5 to 10 $\mu$g/ml ferritin in PBS. After washing, they were blocked by PBS-0.5% BSA for 2 hours at room temperature. $6.25 \times 10^3$, $12.5 \times 10^3$ and $25 \times 10^3$ CPM (200 $\mu$l) of the antibodies were added in the coated tubes. The activity in each tube was counted in the gamma counter, then the tubes were washed three times with PBS-0.4% Tween-20 and the remaining activity counted. The difference between activities with and without the radiolabelled-antibody solutions indicated the percentage of bound-antibody. For evaluation of non specific binding to ferritin, a 100-fold excess of unconjugated antibody was added to the most concentrated radiolabelled-antibody solution. Non specific binding of the antibody was assessed on uncoated tubes. Results showed that regardless of the radionuclide (indium or yttrium), the DTPA-AMB8LK compound had the highest reactivity with ferritin of the three immunoconjugates whereas the DOTA-conjugate had the lowest (Table 1 below). These binding percentages were calculated taking into account small differences in the labelling efficiencies of the three compounds on the day of the experiment.

**Example 7- Immunoreactivity of the radiolabelled-immunoconjugates on CAPAN-1 cells**

Immunoreactivity of the immunoconjugates was assessed on cells expressing ferritin using the method described by Lindmo et al Determination of the immunoreactive fraction of radiolabeled monoclonal antibodies by linear extrapolation to binding at infinite antigen excess. J Immunol Methods 1984;72 (1):77-89.

The CAPAN-1 cells are a relevant model for human pancreatic cancer (Kyriazis et al Human pancreatic adenocarcinoma line Capan-1 in tissue culture and the nude mouse:morphologic, biologic, and biochemical characteristics. Am J Pathol 1982; 106(2):250-60.). This cell line was established from a liver metastasis of a human pancreatic ductal adenocarcinoma, is tumorigenic and metastatic in nude mice and over-expresses ferritin. The $F(ab)_2$ fragment of AMB8LK has been shown to bind to CAPAN-1(Goldstein et al., The design and evaluation of a novel targeted drug delivery system using cationic emulsion-antibody conjugates. J Control Release 2005:108 (2-3):418-32).

CAPAN-1 cells were grown in RPMI, 10% foetal calf serum and 2 mM L-glutamine. Cells were harvested at 70-85% confluence using trypsin, washed and resuspended in PBS-0.5% BSA. They were then fixed and permeabilized using the Intrastain kit (Dako) following the manufacturer's instructions. Cells were diluted in PBS-0.5% BSA at concentrations ranging from $1.25 \times 10^5$ to $12 \times 10^6$ cells/ml and 500 $\mu$l of these suspensions were incubated with a fixed amount of $^{111}$In-labeled-Ab (250 $\mu$l, 50 ng/ml, approximately 100,000CPM) for 2 hours at room temperature under agitation. Suspensions were centrifuged at 5,000 $\times$ rpm for 5 minutes, the cells were washed with PBS-BSA and the activity in the pellets was counted. Nonspecific binding was determined by co-incubation of the radiolabelled-antibody with a 1,000-fold excess of cold antibody. The total radioactive counts added to each tube were divided by the cell-bound counts after substraction of the non-specifically bound counts. These values were plotted against the reciprocal of cell dilution. After linear regression analysis, the immmunoreactive fraction was obtained from the reciprocal of the intercept on the y-axis.

Assays, using non-fixed non-permeabilized CAPAN-1 cells, failed to show binding of the conjugates. However, if the cells were fixed and permeabilized before incubation with the antibody, high levels of specific binding were achieved. The experiments demonstrated that, like pure ferritin, the reactivity of the DTPA conjugate was greater than that of the CHX-DTPA while the DOTA-AMB8LK had the least reactivity on the CAPAN-1 cells (Table 1 below).

**Table 1 : Immunoreactivity of conjugated AMB8LK on ferritin**

| | Pure ferritin[a] | | Cells[b] |
|---|---|---|---|
| | $^{111}$In | $^{90}$Y | $^{111}$In |
| Bz-DTPA-AMB8LK | 87.0% | 100% | 52% |
| Bz-CHX-A"-DTPA AMB8LK | 74.8% | 98.9% | 43% |
| Bz-DOTA-AMB8LK | 76.8% | 73.2% | 24% |

[a] Ferritin was immobilised at 5 $\mu$g/ml on tubes, then the indium or yttrium conjugated antibodies were incubated at three dilutions (3.75 to 15 $\times$ $10^6$ CPM) Results are expressed as the mean of the percentage of bound antibody after washing the tubes. Non specific binding of the antibodies were less than 2%.

[b] Five serial dilutions of fixed and permeabilized CAPAN-1 cells were incubated with the three radiolabelled compounds. After washing, the activity bound to the cells was counted in a gamma counter. Results were analysed using the GraphPad software; linear regression analysis of (Total CPM/Bound CPM) = f(cells dilution) were very accurate with $r^2$ > 0.99 for all three conjugates.

**Example 8-Biodistribution of the indium- and yttrium-radiolabelled conjugated antibodies in normal and tumor bearing mice**

All animal studies were performed in compliance with the UK Animals (Scientific Procedures) Act of 1986 and the Code of Practice for the Housing and Care of Animals used in Scientific Procedures (Home Office, UK).

A. Biodistribution on non tumour bearing mice

12 female Balb/c mice were injected intravenously with 0.2 MBq of $^{111}$In labelled DTPA-AMB8LK, CHX-DTPA-AMB8LK or DOTA-AMB8LK (1.5 $\mu$g) in 50 $\mu$l of PBS. Groups of 4 animals were sacrificed by cervical dislocation after 24 hours, 48 hours and 72 hours. A sample of their blood, femur and muscle were dissected and weighed. Liver, lung, stomach, spleen, intestine and kidneys were also collected. The activity in each sample was counted in a gamma counter together with dilutions of the injected $^{111}$In-Ab. The uptake was expressed as a percentage of the injected dose per gram of tissue for the blood, bone and muscle, while for all other organs, the activity was expressed as a percentage of the injected dose per organ.

As can be seen in Table 2, the liver showed the highest uptake of all the organs, with an uptake of the CHX-DTPA- and the DOTA-AMB8LK of 13-15% of the injected dose (ID), while that of the DTPA-AMB8LK was 7-8%. Other organs showing significant levels of uptake were the bones, especially by the CHX-DTPA-conjugate (4.5$\pm$1.5% of the ID after 24 hours; 6.4$\pm$2.0% after 72 hours) and the intestine (<4% of the ID for the three conjugates) (Table 2). Uptake in the muscle, lung and kidneys was less than 2%; that in the stomach and spleen less than 1%. Circulating activity in the blood was the lowest for DOTA-AMB8LK (5.7$\pm$3.9% of the ID/g after 24 hours and 3.1$\pm$2.1% after 72 hours); the activities of the two DTPA conjugates in the blood were similar after 24h but the subsequent clearance of the CHX-DTPA-AMB8LK was faster than that of the DTPA-AMB8LK (Table 2 below). Based on these results, the DTPA-AMB8LK seemed to be the most promising of the three immunoconjugates despite its relatively slow blood clearance (still 12.6$\pm$0.8% of the ID/g 72 hours after injection).

**Table 2: Biodistribution of $^{111}$In-labelled Bz-DTPA-, Bz-CHX-A'-DTPA and Bz-DOTA-AMB8LK in normal mice**

| | ***Bz-DTPA-AMB8LK Mean ± SD, n=4*** | | | ***Bz-CHX-A"-DTPA-AMB8LK Mean ± SD, n=4*** | | | ***Bz-DOTA-AMB8LK Mean ± SD, n=4*** | | |
|---|---|---|---|---|---|---|---|---|---|
| | **24h** | **48h** | **72h** | **24h** | **48h** | **72h** | **24h** | **48h** | **72h** |
| *Blood* | 15.8 ± 1.2 | 13.3 ± 1.8 | 12.6 ± 0.8 | 16.7 ± 1.6 | 12.3 ± 1.2 | 9.7 ± 1.9 | 5.7 ± 3.9 | 5.6 ± 1.1 | 3.1 ± 2.1 |

(continued)

| | Bz-DTPA-AMB8LK Mean ± SD, n=4 | | | Bz-CHX-A"-DTPA-AMB8LK Mean ± SD, n=4 | | | Bz-DOTA-AMB8LK Mean ± SD, n=4 | | |
|---|---|---|---|---|---|---|---|---|---|
| | 24h | 48h | 72h | 24h | 48h | 72h | 24h | 48h | 72h |
| *Bone* | 2.1 ± 0.4 | 2.6 ± 0.2 | 1.7 ± 1.4 | 4.5 ± 1.5 | 4.4 ± 2.6 | 6.4 ± 2.0 | 1.6 ± 1.3 | 2.3 ± 0.3 | 1.8 ± 0.7 |
| *Muscle* | 1.4 ± 0.2 | 1.3 ± 0.2 | 1.0 ± 0.4 | 1.4 ± 0.1 | 1.2 ± 0.2 | 1.1 ± 0.1 | 0.6 ± 0.4 | 0.6 ± 0.1 | 0.5 ± 0.2 |
| *Liver* | 7.0 ± 0.5 | 7.1 ± 1.0 | 7.5 ± 0.5 | 13.5 ± 1.0 | 13.9 ± 2.1 | 12.9 ± 1.9 | 13.6 ± 7.8 | 15.1 ± 2.5 | 14.4 ± 1.1 |
| *Lung* | 1.2 ± 0.2 | 1.3 ± 0.3 | 1.2 ± 0.3 | 1.7 ± 1.0 | 1.2 ± 0.3 | 1.0 ± 0.5 | 0.7 ± 0.5 | 0.6 ± 0.1 | 0.4 ± 0.3 |
| *Stomach* | 0.4 ± 0.1 | 0.3 ± 0.1 | 0.3 ± 0.1 | 0.3 ± 0.0 | 0.3 ± 0.0 | 0.2 ± 0.1 | 0.1 ± 0.1 | 0.2 ± 0.1 | 0.1 ± 0.1 |
| *Spleen* | 0.5 ± 0.0 | 0.5 ± 0.1 | 0.5 ± 0.1 | 0.7 ± 0.0 | 0.7 ± 0.1 | 0.7 ± 0.1 | 0.4 ± 0.2 | 0.5 ± 0.1 | 0.5 ± 0.2 |
| *Intestine* | 3.2 ± 0.2 | 3.7 ± 0.8 | 2.9 ± 0.3 | 3.9 ± 0.1 | 3.5 ± 0.3 | 2.9 ± 0.3 | 1.7 ± 0.6 | 2.3 ± 0.2 | 1.6 ± 0.5 |
| *Kidneys* | 1.5 ± 0.1 | 1.3 ± 0.1 | 1.1 ± 0.1 | 1.8 ± 0.1 | 1.4 ± 0.2 | 1.1 ± 0.1 | 0.7 ± 0.4 | 0.7 ± 0.1 | 0.4 ± 0.1 |

**B. *In vivo* tumour model: biodistribution on CAPAN-1 tumours bearing mice**

Tumours were initially established in donor mice by sub-cutaneous injection of 0.5 × $10^6$ CAPAN-1 cells in 100 $\mu$l of PBS into the flank of nu/nu mice (Cancer Research-UK, London). After a 5 month period, the tumours were minced and transplanted into 24 nu/nu mice and allowed to grow for 15 to 21 days. Mice were injected i.v. with the radiolabelled antibodies: 0.2 MBq (1.5 $\mu$g) for the $^{111}$In- and 0.4 MBq (3 $\mu$g) for the $^{90}$Y-labelled antibodies, 50 $\mu$l/PBS. At selected time points, groups of 4 animals were killed by cervical dislocation and the organs were resected: samples of blood, bone, and muscle and the entire tumour, liver, lung, stomach, spleen, intestine, kidneys and pancreas. The data was processed as described above for the non-tumour bearing mice.

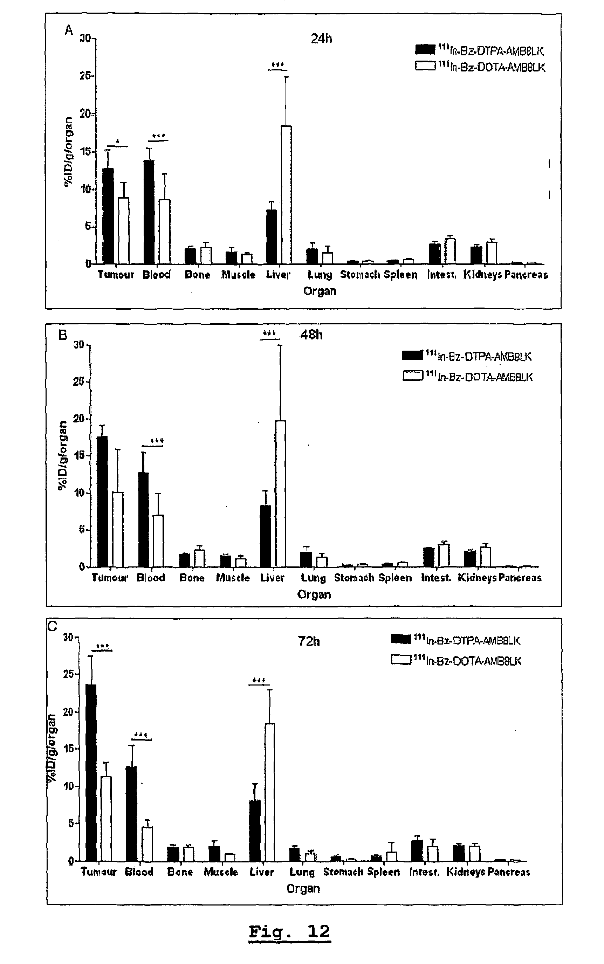

Tumour uptake of $^{111}$In-DTPA-AMB8LK was 12.8±2.4% of the injected dose (ID) per gram of organ 24 hours after the injection. After 72 hours it reached 23.6±3.9% (Table 3 and Fig. 12). The tumour targeting of the $^{111}$In-labelled-DOTA conjugate was inferior to that of the DTPA: starting from 8.9±2.0% after 24 hours, it was only 11.2±1.9% of the ID/g after three days. As for the $^{90}$Y-labelled DTPA-conjugate, uptake by the tumour was 14.0±7.5% of the ID/g after 24 hours. It peaked at 18.6±1.9% after 48 hours, then declined to 16.2±2.9% at the last time point of this experiment, 120 hours (Table 4 and Fig.13). Tumour uptake of $^{90}$Y-DOTA-AMB8LK was 14.1±1.2% at 24 hours and declined to 11.2±4.5% after 120h.

Liver uptake of both compounds, labelled with indium or yttrium, was very similar to that obtained in non-tumour bearing mice. It showed again a liver uptake of the DOTA-conjugate more than twice as high as that of DTPA (Tables 3 and 4 and Figs. 12 and 13).

**Table 3: Biodistribution of $^{111}$In-labelled Bz-DTPA- and Bz-DOTA-AMB8LK in CAPAN-1 tumour bearing mice.**

| | Bz-DTPA-AMB8LK Mean ± SD, n=4 | | | Bz-DOTA-AMB8LK Mean ± SD, n=4 | | |
|---|---|---|---|---|---|---|
| | 24h | 48h | 72h | 24h | 48h | 72h |
| *Blood* | 14.0 ± 1.6 | 12.7 ± 2.8 | 12.7 ± 2.8 | 8.6 ± 3.4 | 7.0 ± 3.0 | 4.5 ± 1.0 |
| *Bone* | 2.0 ± 0.4 | 1.8 ± 0.2 | 1.8 ± 0.4 | 2.3 ± 0.6 | 2.2 ± 0.6 | 1.8 ± 0.3 |
| *Muscle* | 1.7 ± 0.5 | 1.6 ± 0.2 | 2.0 ± 0.7 | 1.3 ± 0.2 | 1.2 ± 0.4 | 0.9 ± 0.1 |
| *Tumour* | 12.8 ± 2.4 | 17.6 ± 1.5 | 23.6 ± 3.9 | 8.9 ± 2.0 | 12.6 ± 3.9 | 11.2 ± 1.9 |
| *Liver* | 7.2 ± 1.2 | 8.4 ± 2.0 | 8.2 ± 2.1 | 18.6 ± 6.3 | 19.7 ± 10.2 | 18.4 ± 4.7 |
| *Lung* | 2.0 ± 0.9 | 2.1 ± 0.7 | 1.6 ± 0.4 | 1.5 ± 1.0 | 1.4 ± 0.5 | 1.0 ± 0.4 |
| *Stomach* | 0.4 ± 0.1 | 0.3 ± 0.1 | 0.5 ± 0.2 | 0.4 ± 0.1 | 0.3 ± 0.1 | 0.3 ± 0.1 |

(continued)

| | Bz-DTPA-AMB8LK Mean ± SD, n=4 | | | Bz-DOTA-AMB8LK Mean ± SD, n=4 | | |
|---|---|---|---|---|---|---|
| | **24h** | **48h** | **72h** | **24h** | **48h** | **72h** |
| *Spleen* | 0.6 ± 0.1 | 0.4 ± 0.1 | 0.6 ± 0.2 | 0.6 ± 0.1 | 0.6 ± 0.1 | 1.2 ± 1.2 |
| *Intestine* | 2.6 ± 0.4 | 2.5 ± 0.1 | 2.7 ± 0.1 | 3.4 ± 0.4 | 3.1 ± 0.4 | 2.0 ± 1.0 |
| *Kidneys* | 2.3 ± 0.2 | 2.1 ± 0.2 | 2.1 ± 0.3 | 2.9 ± 0.5 | 2.7 ± 0.5 | 2.0 ± 0.3 |
| *Pancreas* | 0.2 ± 0.0 | 0.1 ± 0.0 | 0.1 ± 0.0 | 0.2 ± 0.0 | 0.1 ± 0.0 | 0.1 ± 0.0 |

Values are presented as the mean percentage of the injected dose per organ (or per g of tissue for the blood, the bone, the muscle and the tumour) ± standard deviation, n=4 for each group.

**Table 4: Biodistribution of $^{90}$Y-labelled Bz-DTPA- and Bz-DOTA-AMB8LK in CAPAN-1 tumour bearing mice.**

| | Bz-DTPA-AMB8LK Mean ± SD, n=4 | | | Bz-DOTA-AMB8LK Mean ± SD, n=4 | | |
|---|---|---|---|---|---|---|
| | **24h** | **48h** | **120h** | **24h** | **48h** | **120h** |
| *Blood* | 22.3 ± 2.4 | 23.6 ± 5.3 | 15.4 ± 0.8 | 18.5 ± 10.0 | 11.0 ± 1.1 | 8.7 ± 3.5 |
| *Bone* | 5.1 ± 1.1 | 4.4 ± 0.4 | 5.2 ± 0.8 | 3.8 ± 0.5 | 3.4 ± 0.9 | 2.7 ± 0.8 |
| *Muscle* | 2.1 ± 0.4 | 1.7 ± 0.3 | 1.9 ± 0.4 | 1.4 ± 0.2 | 1.2 ± 0.2 | 1.1 ± 0.3 |
| *Tumour* | 14.0 ± 7.5 | 18.6 ± 1.9 | 16.2 ± 2.9 | 14.1 ± 1.2 | 12.9 ± 2.3 | 11.2 ± 4.5 |
| *Liver* | 6.9 ± 0.6 | 6.7 ± 0.4 | 6.4 ± 0.3 | 13.6 ± 2.8 | 14.5 ± 4.5 | 11.8 ± 1.8 |
| *Lung* | 2.7 ± 1.0 | 2.1 ± 0.6 | 2.2 ± 0.6 | 1.4 ± 0.5 | 1.3 ± 0.4 | 1.0 ± 0.2 |
| *Stomach* | 0.5 ± 0.1 | 0.6 ± 0.2 | 0.3 ± 0.1 | 0.3 ± 0.2 | 0.3 ± 0.1 | 0.2 ± 0.1 |
| *Spleen* | 0.8 ± 0.0 | 0.7 ± 0.1 | 0.8 ± 0.1 | 0.7 ± 0.2 | 0.8 ± 0.1 | 0.7 ± 0.1 |
| *Intestine* | 2.9 ± 0.7 | 1.9 ± 0.0 | 1.6 ± 0.2 | 1.7 ± 0.3 | 1.6 ± 0.3 | 1.0 ± 0.1 |
| *Kidneys* | 3.4 ± 0.2 | 3.4 ± 0.3 | 2.8 ± 0.1 | 1.8 ± 0.2 | 1.6 ± 0.2 | 1.2 ± 0.1 |
| *Pancreas* | 0.4 ± 0.3 | 0.1 ± 0.0 | 0.1 ± 0.0 | 0.1 ± 0.0 | 0.1 ± 0.1 | 0.1 ± 0.1 |

Values are presented as the mean percentage of the injected dose per organ (or per g of tissue for the blood, the bone, the muscle and the tumour) ± standard deviation, n=4 for each group.

The uptake of the radiolabelled antibodies in the stomach, spleen and pancreas was very low (less than 1% of the ID). Although the bone uptake was <5% of the ID for the two conjugates at all time points, significant differences were noticed between the uptake of the indium and yttrium-labelled-DTPA-antibody: the bone uptake of the $^{90}$Y-DTPA-AMB8LK was greater than that of the $^{111}$In-DTPA-AMB8LK at 24 hours and 48 hours time points (P<0.001). No such difference occurred between the yttrium- and indium-labelled-DOTA-antibody: P<0.05 at 24 hours but no significant difference at 48 hours.

### *C.* Imaging of $^{111}$In-DTPA-AMB8LK in mouse

One CAPAN-1 tumour bearing mouse was injected i.v. with 20 MBq of $^{111}$In-labelled DTPA-AMB8LK. At required time points, the mouse was anesthetised by ketamine/xylazine (2/1) injection then imaged during 20 minutes with a NanoSPECT/CT apparatus (Bioscan). The SPECT reconstructions had a voxel size of 0.4 mm, the CT of 0.2 mm.

Figure 14 shows some images, wherein the scale of the radioactivity levels and some of the organs are indicated. Decay correction was applied to get all of the studies at the same level since the half-life of indium is 67.2 hours. 1 hour after injection, the majority of the injected compound was located in the bladder and the heart with also important uptake in the liver and lungs. As can be seen and as already observed in the biodistribution experiments reported above, significant tumour accumulation occurred 24 hours after the compound injection was injected with still some localization in the heart and liver. Radioactivity in the tumour is still present after 72 hours.

### D.Statistical analysis

Biodistribution data were analysed with the GraphPad Prism software (San Diego, *CA*). Data are expressed as mean ± 5D and evaluated for statistical significance with two-way ANOVA followed by a Bonferroni post test. The criteria of significance were set as * P<0.05, ** P<0.01 and ***P<0.001.

**Example 9- *In vitro* assessment of the *ADCC* of chimeric AMB8LK**

The *ADCC* activity of the chimeric AMB8LK antibody was measured on the non-labeled antibody set forth above, using the following protocol: CAPAN-1 cells were stained with carboxyfluorescein diactete succinimidyl ester, which is a reagent for the analysis of cellular proliferation. Basically, peripheral blood mononuclear cells were isolated from heparinized blood following the ACTG PBMC Consensus Method. The cells were harvested from the interface of a ficoll layer and were washed twice with RPMI 1640 medium. After the last wash, the cells were transferred to a test tube and a viable cell count was undertaken. The cells were then spun at 800 $\times$ g for 10 minutes and the RPMI medium was removed. The cells were then resuspended in 2.5 ml of a 0.1% BSA/PBS solution at room temperature, then were centrifuged at 800 $\times$ g for 10 minutes and the supernatant was decanted. 250 $\mu$l of a 0.1% to 0.5 % BSA in PBS was added to the cell pellet and the cells were then suspended.

Capan-1 cells were then stained with diluted CFSE (CarboxyFluorescein diacetate Succinimidyl Ester) for 15 min at 37°C. Media was then added in a large excess to complex the unbound CFSE. Then, cells were washed 3 times with PBS and resuspended in complete media (1 ml/$10^6$ cells) that is RPMI, 10% human AB sera, 1% L-Glutamine, 1% Hepes buffer and 1% penicillin/streptomycin. 1 ml of cells were used per well of a 24-well plate. Addition of PBMC at a PBMC:cells ratio ranging from 5:1 to 50:1 and of the AMB8LK antibody (1 to 10 $\mu$g/well by example) was done. The cells were centrifuged at low speed (1000rpm) and then left to incubate for 30 min at 37°C. The 7AAD (7-amino-actinomycin D) stain was then added and the cells were analysed on a cell cytometer. Results showed that the AMB8LK antibody has ADCC activity on Capan-1 cells.

The same experiments may be carried out with other chimeric monoclonal antibody or with antibody-like molecules disclosed in the present specification, to assess *in vitro* ADCC activity.

**Example 10-therapeutic Effects on tumor growth using radiolabelled chimeric monoclonal AMB8LK antibodies.**

Monoclonal antibodies AMB8LK were produced as set forth above in Example I, conjugated as in Example III and radiolabeled as in Example IV above.

<u>LD50 determination</u>

Firstly, a radioactive dose escalation study to determine the $LD_{50}$ is done using 6 groups of 4 mice: chimeric AMB8LK antibody is radiolabeled using yttrium-90 at various specific activities, so as to inject in each mouse the same antibody amount: 49MBq of $^{90}$Y/mg of antibody, 86MBq of $^{90}$Y/mg of antibody, 123MBq of $^{90}$Y/mg of antibody, 185MBq of $^{90}$Y/mg of antibody and 246MBq of $^{90}$Y /mg of antibody. Then, group I mice are injected with 30$\mu$g of cold antibody, group II mice with 30$\mu$g of chimeric AMB8LK radiolabeled at a specific activity of 49MBq of $^{90}$Y/mg of antibody (40$\mu$Ci/mouse), group III mice with 30$\mu$g of chimeric AMB8LK radiolabeled at a specific activity of 86MBq of $^{90}$Y/mg of antibody (70$\mu$Ci/mouse), group IV mice with 30$\mu$g of chimeric AMB8LK radiolabeled at a specific activity of 123MBq of $^{90}$Y/mg of antibody (100$\mu$Ci/mouse), group V mice with 30$\mu$g of chimeric AMB8LK radiolabeled at a specific activity of 185MBq of $^{90}$Y/mg of antibody (150$\mu$CI/mouse) and group VI with 30 $\mu$g of chimeric AMB8LK radiolabeled at a specific activity of 246MBq of $^{90}$Y/mg of antibody (200$\mu$Ci/mouse). Mice are weighed twice a week, during 8 weeks after the injection and tumour size is recorded twice a week. If a mouse dies during the observation period, an autopsy is performed to determine if the death is due to radiotoxicity.

<u>Tumor volume reduction</u>

Once the $LD_{50}$ is established, the therapeutic efficacy of the chimeric AMB8LK antibody can be studied using four specific activities, all below the $LD_{50}$. 5$\times$$10^6$ CAPAN-1 cells are injected into Balb/c/nu/nu mice as described in a previous example to establish tumour in Balb/c/nu/nu mice. Once the tumour reaches the volume of 200 mm$^3$, experiments can begin. Four groups of 6 mice are monitored for 84 days and are sacrificed, if the tumor has a volume of >1000 mm$^3$.

Group I-mice are administered with 30 $\mu$g of the chimeric monoclonal antibody of AMB8LK of the present invention, radiolabeled with yttrium at a specific activity n°1.

Group II-mice are injected with 30 $\mu$g of the chimeric monoclonal antibody of AMB8LK of the present invention, radiolabeled with yttrium at a specific activity n°2.

Group III-mice are injected with 30 $\mu$g of the chimeric monoclonal antibody of AMB8LK of the present invention, radiolabeled with yttrium at a specific activity n°3.

Group IV-mice are injected with 30 $\mu$g of the chimeric monoclonal antibody of AMB8LK of the present invention, radiolabeled with yttrium at a specific activity n°4.

The above experiment shows that there is effective tumor reduction using the radiolabeled antibodies of the present invention and *in vitro* ADCC activity of the AMB8LK antibody.

The same experiments may be carried out with other chimeric monoclonal antibody or with antibody-like molecules as disclosed in the present specif ication.

## Claims

1. A chimeric anti-ferritin monoclonal antibody, that binds both human acidic and basic ferritin, comprising:

   (a) two heavy chain polypeptides, each comprising the VH domain defined in SEQ ID NO:4; and
   (b) two light chain polypeptides, each comprising the VL domain as defined in SEQ ID NO:2.

2. The chimeric anti-ferritin monoclonal antibody according to claim 1, wherein each of said heavy chains further comprises a CH1 constant region, optionally comprising a human Fc region or a murine Fc region, and/or a signal peptide.

3. The chimeric anti-ferritin monoclonal antibody according to claim 2, wherein said signal peptide comprises the amino acid sequence set forth in SEQ ID NO:16.

4. The chimeric anti-ferritin monoclonal antibody according to claim 1, wherein each of said light chains further comprises a constant kappa or lambda region and/or a signal peptide.

5. The chimeric anti-ferritin monoclonal antibody according to claim 4, wherein said constant kappa or lambda region comprises the amino acid sequence set forth in SEQ ID NO:12.

6. The chimeric anti-ferritin monoclonal antibody according to claim 4, wherein said signal peptide comprises the amino acid sequence set forth in SEQ ID NO:14.

7. The chimeric anti-ferritin monoclonal antibody according to any one of claims 1 to 6, that comprises as a light chain, from the N-terminal part to the C-terminal part, SEQ ID NO:2 and SEQ ID NO:12, and as a heavy chain, from the N-terminal part to the C-terminal part, SEQ ID NO: 4, SEQ ID NO:6 and a human Fc region.

8. A functional fragment of the antibody according to any one of claims 1 to 7, wherein said functional fragment is a Fv fragment or a Fab fragment and wherein said functional fragment specifically binds both human acid and basic ferritins.

9. A bispecific or trispecific monoclonal antibody comprising at least one Fab fragment according to claim 8, wherein at least one Fab fragment consists of a polypeptide consisting from the N-terminal to the C-terminal of SEQ ID NO:2 directly linked to SEQ ID NO:12 and a polypeptide consisting from the N-terminal to the C-terminal of SEQ ID NO:4 directly linked to SEQ ID NO:6.

10. The functional fragment according to claim 8, wherein said fragment is linked to a ligand.

11. The functional fragment according to claim 10, wherein said ligand is a cytokine, a receptor or any protein of interest.

12. A scFv molecule consisting of a VH domain as defined in SEQ ID NO:4, linked to a VL domain as defined in SEQ ID NO:2, wherein said scFv molecule binds both human acidic and basic ferritins.

13. A diabody molecule comprising at least a VH domain as defined in SEQ ID NO:4 and at least a VL domain as defined in SEQ ID NO:2, wherein said diabody molecule binds both human acidic and basic ferritins.

14. A murine anti-ferritin monoclonal antibody that binds both human acidic and basic ferritin comprising as a light chain, from the N-terminal part to the C-terminal part, SEQ ID NO:2 and SEQ ID NO:12, and as a heavy chain, from the N-terminal part to the C-terminal part, SEQ ID NO:4, SEQ ID NO:6 and a murine Fc region.

15. A molecule selected from the group consisting of a chimeric anti-ferritin monoclonal antibody, a functional fragment, a bispecific monoclonal antibody, a trispecific monoclonal antibody and a scFv molecule according to any one of claims 1 to 13, a Bis-scFv comprising at least one scFv according to claim 12 and a diabody according to claim 13, further comprising a radioisotope conjugated thereto.

**16.** The molecule according to claim 14, wherein said radioisotope is an alpha-, beta-, gamma-, beta-gamma- or alpha-beta-emitting radioisotope.

**17.** A molecule selected from the group consisting of a chimeric anti-ferritin monoclonal antibody, a functional fragment, a bispecific monoclonal antibody, a trispecific monoclonal antibody and a scFv molecule according to any one of claims 1 to 13, a Bis-scFv comprising at least one scFv according to claim 12 and a diabody according to claim 13, further comprising a drug conjugated thereto.

**18.** The molecule according to claim 17, wherein said drug is a pyrimidine drug.

**19.** A pharmaceutical composition comprising a molecule selected from the group consisting of a chimeric or human anti-ferritin monoclonal antibody according to any one of claims 1 to 7 a functional fragment according to claim 8, a bispecific or trispecific monoclonal antibody according to claim 9, a scFv molecule according to claim 12, a Bis-scFv comprising a scFv molecule according to claim 12 and a diabody according to claim 13, and a pharmaceutically acceptable vehicle.

**20.** The composition according to claim 19, in a pharmaceutically acceptable carrier, wherein a drug, radioactive materials, toxins, immune killer cells or combinations thereof is attached through a chelating agent or a linker to said chimeric anti-ferritin monoclonal antibody, functional fragment, bispecific or trispecific monoclonal antibody, scFv molecule, Bis-scFv or diabody, for use as a therapeutic agent.

**21.** The composition according to claim 19, in a pharmaceutically acceptable carrier for administration simultaneously or at specific intervals with a composition selected from the group of a drug, radioactive materials, toxins, immune killer cells and combinations thereof, in the treatment of a cancer selected from the group consisting of pancreatic cancer, Hodgkins lymphoma, Kaposi sarcoma and hepatocellular carcinoma.

**22.** A vector comprising a nucleic acid encoding a heavy chain according to any one of claims 1 to 7 and a nucleic acid encoding a light chain according to any one of claims 1 to 7, and optionally comprising a promoter for baculovirus expression or a eukaryotic promoter.

**23.** The vector according to claim 22, wherein said nucleic acid encoding the heavy chain comprises SEQ ID NO:3 and wherein said nucleic acid encoding the light chain comprises SEQ ID NO:1.

**24.** Cells transformed with a vector according to any one of claims 22 or 23 wherein said cells are selected from the group consisting of CHO cells, *E. coli,* yeast cells, VERO cells, HELA cells, COS cells, CR cells: 1650, W138, BHK, HepG2, 3T3, A549, PC12, K562, 293 cells, Sf9 cells and Cv1 cells.

**25.** Cells transformed with a vector comprising a nucleic acid encoding a heavy chain according to any one of claims 1 to 7 and with a vector comprising a nucleic acid encoding a light chain according to any one of claims 1 to 7, in particular selected from the group consisting of CHO cells, *E. coli,* yeast cells, VERO cells, HELA cells, COS cells, CR cells: 1650, W138, BHK, HepG2, 3T3, A549, PC12, K562, 293 cells, Sf9 cells and Cv1 cells.

**26.** The cells according to any one of claims 24 to 25, wherein said nucleic acid encoding the heavy chain comprises SEQ ID NO:3, and wherein said nucleic acid encoding the light chain comprises SEQ ID NO:1.

**Patentansprüche**

**1.** Chimärer Anti-Ferritin monoklonaler Antikörper, der sowohl humanes saures als auch basisches Ferritin bindet, umfassend:

(a) zwei schwere Kette-Polypeptide, jeweils umfassend die in SEQ ID NO: 4 definierte VH-Domäne; und
(b) zwei leichte Kette-Polypeptide, jeweils umfassend die wie in SEQ ID NO: 2 definierte VL-Domäne.

**2.** Chimärer Anti-Ferritin monoklonaler Antikörper nach Anspruch 1, wobei jede der schweren Ketten des Weiteren eine CH1-konstante Region umfasst, gegebenenfalls umfassend eine humane Fc-Region oder eine murine Fc-Region, und/oder ein Signalpeptid.

3. Chimärer Anti-Ferritin monoklonaler Antikörper nach Anspruch 2, wobei das Signalpeptid die in SEQ ID NO: 16 dargelegte Aminosäuresequenz umfasst.

4. Chimärer Anti-Ferritin monoklonaler Antikörper nach Anspruch 1, wobei jede der leichten Ketten des Weiteren eine konstante kappa- oder lambda-Region und/ oder ein Signalpeptid umfasst.

5. Chimärer Anti-Ferritin monoklonaler Antikörper nach Anspruch 4, wobei die konstante kappa- oder lambda-Region die in SEQ ID NO: 12 dargelegte Aminosäuresequenz umfasst.

6. Chimärer Anti-Ferritin monoklonaler Antikörper nach Anspruch 4, wobei das Signalpeptid die in SEQ ID NO: 14 dargelegte Aminosäuresequenz umfasst.

7. Chimärer Anti-Ferritin monoklonaler Antikörper nach einem beliebigen der Ansprüche 1 bis 6, der als eine leichte Kette von dem N-terminalen Teil bis zu dem C-terminalen Teil SEQ ID NO: 2 und SEQ ID NO: 12, und als eine schwere Kette von dem N-terminalen Teil bis zu dem C-terminalen Teil SEQ ID NO: 4, SEQ ID NO: 6 und eine humane Fc-Region umfasst.

8. Funktionelles Fragment des Antikörpers gemäß einem beliebigen der Ansprüche 1 bis 7, wobei das funktionelle Fragment ein Fv-Fragment oder ein Fab-Fragment ist und wobei das funktionelle Fragment spezifisch sowohl humane saure als auch basische Ferritine bindet.

9. Bispezifischer oder trispezifischer monoklonaler Antikörper, umfassend mindestens ein Fab-Fragment gemäß Anspruch 8, wobei mindestens ein Fab-Fragment aus einem Polypeptid besteht, bestehend aus vom N-Terminus bis zum C-Terminus von SEQ ID NO: 2, direkt verknüpft mit SEQ ID NO: 12, und einem Polypeptid, bestehend vom N-Terminus bis zum C-Terminus von SEQ ID NO: 4, direkt verknüpft mit SEQ ID NO: 6.

10. Funktionelles Fragment nach Anspruch 8, wobei das Fragment mit einem Liganden verknüpft ist.

11. Funktionelles Fragment nach Anspruch 10, wobei der Ligand ein Cytokin, ein Rezeptor oder ein beliebiges Protein von Interesse ist.

12. Ein scFv-Molekül, bestehend aus einer wie in SEQ ID NO: 4 definierten VH-Domäne, verknüpft mit einer wie in SEQ ID NO: 2 definierten VL-Domäne, wobei das scFv-Molekül sowohl humane saure als auch basische Ferritine bindet.

13. Diabody-Molekül, umfassend mindestens eine wie in SEQ ID NO: 4 definierte VH-Domäne und mindestens eine wie in SEQ ID NO: 2 definierte VL-Domäne, wobei das Diabody-Molekül sowohl humane saure als auch basische Ferritine bindet.

14. Muriner Anti-Ferritin monoklonaler Antikörper, der sowohl humanes saures als auch basisches Ferritin bindet, umfassend vom N-terminalen Teil bis zum C-terminalen Teil, SEQ ID NO: 2 und SEQ ID NO: 12 als eine leichte Kette und vom N-terminalen Teil zum C-terminalen Teil, SEQ ID NO: 4, SEQ ID NO: 6 und eine murine Fc-Region als eine schwere Kette.

15. Molekül, ausgewählt aus der Gruppe, bestehend aus einem chimären Anti-Ferritin monoklonalen Antikörper, einem funktionellen Fragment, einem bispezifischen monoklonalen Antikörper, einem trispezifischen monoklonalen Antikörper und einem scFv-Molekül gemäß einem beliebigen der Ansprüche 1 bis 13, ein Bis-scFv, umfassend mindestens ein scFv gemäß Anspruch 12 und ein Diabody gemäß Anspruch 13, des Weiteren umfassend ein daran konjugiertes Radioisotop.

16. Molekül nach Anspruch 14, wobei das Radioisotop ein alpha-, beta-, gamma-, beta-gamma- oder alpha-beta-strahlendes Radioisotop ist.

17. Molekül, ausgewählt aus der Gruppe, bestehend aus einem chimären Anti-Ferritin monoklonalen Antikörper, einem funktionellen Fragment, einem bispezifischen monoklonalen Antikörper, einem trispezifischen monoklonalen Antikörper und einem scFv-Molekül gemäß einem beliebigen der Ansprüche 1 bis 13, einem Bis-scFv, umfassend mindestens ein scFv gemäß Anspruch 12 und ein Diabody gemäß Anspruch 13, des Weiteren umfassend einen daran konjugierten Wirkstoff.

**18.** Molekül nach Anspruch 17, wobei der Wirkstoff ein Pyrimidinwirkstoff ist.

**19.** Pharmazeutische Zusammensetzung, umfassend ein Molekül, ausgewählt aus der Gruppe, bestehend aus einem chimären oder humanen Anti-Ferritin monoklonalen Antikörper gemäß einem beliebigen der Ansprüche 1 bis 7, ein funktionelles Fragment gemäß Anspruch 8, ein bispezifischer oder trispezifischer monoklonaler Antikörper gemäß Anspruch 9, ein scFv-Molekül gemäß Anspruch 12, ein Bis-scFv, umfassend ein scFv-Molekül gemäß Anspruch 12 und ein Diabody gemäß Anspruch 13, und ein pharmazeutisch verträgliches Vehikel.

**20.** Zusammensetzung nach Anspruch 19, in einem pharmazeutisch verträglichen Träger, wobei ein Wirkstoff, radioaktive Materialien, Toxine, Immun-Killerzellen oder Kombinationen davon durch ein Chelatisierungsmittel oder einen Linker an den chimären Anti-Ferritin monoklonalen Antikörper, das funktionelle Fragment, den bispezifischen oder trispezifischen monoklonalen Antikörper, das scFv-Molekül, das Bis-scFv oder den Diabody angehängt ist, zur Verwendung als ein therapeutisches Mittel.

**21.** Zusammensetzung nach Anspruch 19, in einem pharmazeutisch verträglichen Träger zur gleichzeitigen Verabreichung oder in spezifischen Intervallen mit einer Zusammensetzung, ausgewählt aus der Gruppe eines Wirkstoffs, radioaktiver Materialien, Toxine, Immun-Killerzellen und Kombinationen davon, bei der Behandlung eines Krebs, ausgewählt aus der Gruppe, bestehend aus Pankreaskrebs, Hodgkins-Lymphom, Kaposi-Sarkom und hepatozellulärem Karzinom.

**22.** Vektor, umfassend eine Nukleinsäure, kodierend eine schwere Kette gemäß einem beliebigen der Ansprüche 1 bis 7, und eine Nukleinsäure, kodierend eine leichte Kette, gemäß einem beliebigen der Ansprüche 1 bis 7, und gegebenenfalls umfassend einen Promotor zur Baculovirus-Expression oder einen eukaryotischen Promotor.

**23.** Vektor nach Anspruch 22, wobei die die schwere Kette kodierende Nukleinsäure SEQ ID NO: 3 umfasst und wobei die die leichte Kette kodierende Nukleinsäure SEQ ID NO: 1 umfasst.

**24.** Zellen, transformiert mit einem Vektor gemäß einem beliebigen der Ansprüche 22 oder 23, wobei die Zellen ausgewählt sind aus der Gruppe, bestehend aus CHO-Zellen, E. coli, Hefezellen, VERO-Zellen, HELA-Zellen, COS-Zellen, CR-Zellen: 1650, W138, BHK, HepG2, 3T3, A549, PC12, K562, 293-Zellen, Sf9-Zellen und Cv1-Zellen.

**25.** Zellen, transformiert mit einem Vektor, umfassend eine Nukleinsäure, kodierend eine schwere Kette gemäß einem beliebigen der Ansprüche 1 bis 7, und mit einem Vektor, umfassend eine Nukleinsäure, kodierend eine leichte Kette gemäß einem beliebigen der Ansprüche 1 bis 7, insbesondere ausgewählt aus der Gruppe, bestehend aus CHO-Zellen, E. coli, Hefezellen, VERO-Zellen, HELA-Zellen, COS-Zellen, CR-Zellen: 1650, W138, BHK, HepG2, 3T3, A549, PC12, K562, 293-Zellen, Sf9-Zellen und Cv1-Zellen.

**26.** Zellen nach einem beliebigen der Ansprüche 24 bis 25, wobei die die schwere Kette kodierende Nukleinsäure SEQ ID NO: 3 umfasst und wobei die die leichte Kette kodierende Nukleinsäure SEQ ID NO: 1 umfasst.

## Revendications

**1.** Anticorps monoclonal anti-ferritine chimérique, qui se lie à la fois à la ferritine humaine acide et basique, comprenant :

(a) deux polypeptides de chaîne lourde, comprenant chacun le domaine VH défini dans la SEQ ID NO: 4 ; et
(b) deux polypeptides de chaîne légère, comprenant chacun le domaine VL tel que défini dans la SEQ ID NO: 2.

**2.** Anticorps monoclonal anti-ferritine chimérique selon la revendication 1, dans lequel chacune desdites chaînes lourdes comprend en outre une région constante CH1, comprenant éventuellement une région Fc humaine ou une région Fc murine, et/ou un peptide signal.

**3.** Anticorps monoclonal anti-ferritine chimérique selon la revendication 2, dans lequel ledit peptide signal comprend la séquence d'acides aminés présentée dans la SEQ ID NO: 16.

**4.** Anticorps monoclonal anti-ferritine chimérique selon la revendication 1, dans lequel chacune desdites chaînes légères comprend en outre une région constante kappa ou lambda et/ou un peptide signal.

5. Anticorps monoclonal anti-ferritine chimérique selon la revendication 4, dans lequel ladite région constante kappa ou lambda comprend la séquence d'acides aminés présentée dans la SEQ ID NO: 12.

6. Anticorps monoclonal anti-ferritine chimérique selon la revendication 4, dans lequel ledit peptide signal comprend la séquence d'acides aminés présentée dans la SEQ ID NO: 14.

7. Anticorps monoclonal anti-ferritine chimérique selon l'une quelconque des revendications 1 à 6, qui comprend en tant que chaîne légère, de la partie N-terminale jusqu'à la partie C-terminale, la SEQ ID NO: 2 et la SEQ ID NO: 12, et en tant que chaîne lourde, de la partie N-terminale jusqu'à la partie C-terminale, la SEQ ID NO: 4, la SEQ ID NO: 6 et une région Fc humaine.

8. Fragment fonctionnel de l'anticorps selon l'une quelconque des revendications 1 à 7, dans lequel ledit fragment fonctionnel est un fragment Fv ou un fragment Fab, et dans lequel ledit fragment fonctionnel se lie spécifiquement aux deux ferritines humaines acide et basique.

9. Anticorps monoclonal bispécifique ou trispécifique comprenant au moins un fragment Fab selon la revendication 8, dans lequel au moins un fragment Fab consiste en un polypeptide consistant en la N-terminale à la C-terminale de la SEQ ID NO: 2 directement liée à la SEQ ID NO: 12 et un polypeptide consistant en la N-terminale à la C-terminale de la SEQ ID NO: 4 directement liée à la SEQ ID NO: 6.

10. Fragment fonctionnel selon la revendication 8, dans lequel ledit fragment est lié à un ligand.

11. Fragment fonctionnel selon la revendication 10, dans lequel ledit ligand est une cytokine, un récepteur ou toute protéine d'intérêt.

12. Molécule de scFv constituée d'un domaine VH tel que défini dans la SEQ ID NO: 4, lié à un domaine VL tel que défini dans la SEQ ID NO: 2, dans laquelle ladite molécule de scFv se lie aux deux ferritines humaines acide et basique.

13. Molécule de dianticorps comprenant au moins un domaine VH tel que défini dans la SEQ ID NO: 4 et au moins un domaine VL tel que défini dans la SEQ ID NO: 2, dans lequel ladite molécule de dianticorps se lie aux deux ferritines humaines acide et basique.

14. Anticorps monoclonal murin anti-ferritine qui se lie aux deux ferritines humaines acide et basique, comprenant en tant que chaîne légère, de la partie N-terminale jusqu'à la partie C-terminale, la SEQ ID NO: 2 et la SEQ ID NO: 12, et en tant que chaîne lourde, de la partie N-terminale jusqu'à la partie C-terminale, la SEQ ID NO: 4, la SEQ ID NO: 6 et une région Fc murine.

15. Molécule choisie dans le groupe constitué par un anticorps monoclonal anti-ferritine chimérique, un fragment fonctionnel, un anticorps monoclonal bispécifique, un anticorps monoclonal trispécifique et une molécule de scFv selon l'une quelconque des revendications 1 à 13, un bis-scFv comprenant au moins un scFv selon la revendication 12 et un dianticorps selon la revendication 13, comprenant en outre un radio-isotope conjugué à celle-ci.

16. Molécule selon la revendication 14, dans laquelle ledit radio-isotope est un radio-isotope émettant des particules alpha, bêta, gamma, bêta-gamma ou alpha-bêta.

17. Molécule choisie dans le groupe constitué par un anticorps monoclonal anti-ferritine chimérique, un fragment fonctionnel, un anticorps monoclonal bispécifique, un anticorps monoclonal trispécifique et une molécule de scFv selon l'une quelconque des revendications 1 à 13, un bis-scFv comprenant au moins un scFv selon la revendication 12 et un dianticorps selon la revendication 13, comprenant en outre un médicament conjugué à celle-ci.

18. Molécule selon la revendication 17, dans laquelle ledit médicament est un médicament de type pyrimidine.

19. Composition pharmaceutique comprenant une molécule choisie dans le groupe constitué par un anticorps monoclonal anti-ferritine chimérique ou humain selon l'une quelconque des revendications 1 à 7, un fragment fonctionnel selon la revendication 8, un anticorps monoclonal bispécifique ou trispécifique selon la revendication 9, une molécule de scFv selon la revendication 12, un bis-scFv comprenant une molécule de scFv selon la revendication 12 et un dianticorps selon la revendication 13, et un véhicule pharmaceutiquement acceptable.

**20.** Composition selon la revendication 19, dans un support pharmaceutiquement acceptable, dans laquelle un médicament, des matériaux radioactifs, des toxines, des cellules tueuses immunitaires ou des combinaisons de ceux-ci, est attaché au moyen d'un agent chélatant ou d'un agent de liaison audit anticorps monoclonal anti-ferritine chimérique, fragment fonctionnel, anticorps monoclonal bispécifique ou trispécifique, molécule de scFv, bis-scFv ou dianticorps, pour une utilisation en tant qu'agent thérapeutique.

**21.** Composition selon la revendication 19, dans un support pharmaceutiquement acceptable pour une administration simultanée ou à des intervalles spécifiques avec une composition choisie dans le groupe d'un médicament, de matériaux radioactifs, de toxines, de cellules tueuses immunitaires ou de combinaisons de ceux-ci, dans le traitement d'un cancer choisi dans le groupe constitué par le cancer du pancréas, le lymphome de Hodgkin, le sarcome de Kaposi et le carcinome hépatocellulaire.

**22.** Vecteur comprenant un acide nucléique codant pour une chaîne lourde selon l'une quelconque des revendications 1 à 7 et un acide nucléique codant pour une chaîne légère selon l'une quelconque des revendications 1 à 7, et comprenant éventuellement un promoteur pour une expression dans un baculovirus ou un promoteur eucaryote.

**23.** Vecteur selon la revendication 22, dans lequel ledit acide nucléique codant pour la chaîne lourde comprend la SEQ ID NO: 3 et dans lequel ledit acide nucléique codant pour la chaîne légère comprend la SEQ ID NO: 1.

**24.** Cellules transformées à l'aide d'un vecteur selon l'une quelconque des revendications 22 ou 23, dans lesquelles lesdites cellules sont choisies dans le groupe constitué par les cellules CHO, *E. coli,* les cellules de levure, les cellules VERO, les cellules HELA, les cellules COS, les cellules CR : 1650, W138, BHK, HepG2, 3T3, A549, PC12, K562, les cellules 293, les cellules Sf9 et les cellules Cv1.

**25.** Cellules transformées à l'aide d'un vecteur comprenant un acide nucléique codant pour une chaîne lourde selon l'une quelconque des revendications 1 à 7 et à l'aide d'un vecteur comprenant un acide nucléique codant pour une chaîne légère selon l'une quelconque des revendications 1 à 7, sélectionnées en particulier dans le groupe constitué par les cellules CHO, *E. coli,* les cellules de levure, les cellules VERO, les cellules HELA, les cellules COS, les cellules CR : 1650, W138, BHK, HepG2, 3T3, A549, PC12, K562, les cellules 293, les cellules Sf9 et les cellules Cv1.

**26.** Cellules selon l'une quelconque des revendications 24 à 25, dans lesquelles ledit acide nucléique codant pour la chaîne lourde comprend la SEQ ID NO: 3, et dans lesquelles ledit acide nucléique codant pour la chaîne légère comprend la SEQ ID NO: 1.

```
CAA ATT GTT CTC ACC CAG TCT CCA GCA ATC CTG TCT GCA TCT CTA GGG    48
 Q   I   V   L   T   Q   S   P   A   I   L   S   A   S   L   G     16
                             FR1

GAG GAG ATC ACC CTA ACC TGC AGT GCC AGC TCG AGT GTA ACT TTC ATG    96
 E   E   I   T   L   T   C   S   A   S   S   S   V   T   F   M     32
         FR1                                  CDR1

CAC TGG TAC CAG CAG AAG TCA GGC ACT TCT CCC AAA CTC TTG ATT TAT   144
 H   W   Y   Q   Q   K   S   G   T   S   P   K   L   L   I   Y     48
                             FR2

ACC ACA TCC AAC CTG GCT TCT GGA GTC CCT TCT CGC TTC AGT GGC AGT   192
 T   T   S   N   L   A   S   G   V   P   S   R   F   S   G   S     64
   CDR2                      FR3

GGG TCT GGG ACC TTT TAT TCT CTC ACA ATC AGC AGT GTG GAG GCT GAA   240
 G   S   G   T   F   Y   S   L   T   I   S   S   V   E   A   E     80
                             FR3

GAT GCT GCC GAT TAT TAC TGC CAT CAG TGG AGT AGT TAT CCC ACG TTC   288
 D   A   A   D   Y   Y   C   H   Q   W   S   S   Y   P   T   F     96
       FR3                                CDR3

GGC TCG GGG ACA AAG TTG GAA ATA AAA CGG                           318
 G   S   G   T   K   L   E   I   K   R                            106
                 FR4
```

**Fig. 1**

```
Q   V   Q   L   K   E   S   G   P   G   L   V   A   P   S   Q   S   L     18
CAG GTG CAG CTG AAG GAG TCA GGA CCT GGC CTG GTG GCA CCC TCA CAG AGC CTG   54
                             FR1

S   I   T   C   T   V   S   G   F   S   L   S   R   Y   S   V   H   W     36
TCC ATC ACA TGC ACT GTC TCT GGG TTC TCA TTA TCC AGA TAT AGT GTA CAC TGG   108
         FR1                            CDR1                    FR2

V   R   Q   P   P   G   K   G   L   E   W   L   G   T   I   W   G   G     54
GTT CGC CAG CCT CCA GGA AAG GGT CTG GAG TGG CTG GGA ACG ATA TGG GGT GGT   162
                         FR2                                CDR2

G   S   T   D   Y   N   S   V   L   K   S   R   L   S   I   S   K   D     72
GGA AGC ACA GAC TAT AAC TCA GTT CTC AAA TCC AGA CTG AGC ATC AGC AAG GAC   216
   CDR2                                 FR3

N   S   K   S   Q   V   L   L   K   V   N   S   L   Q   T   D   D   T     90
AAC TCC AAG AGC CAA GTT TTG TTA AAA GTG AAC AGT CTA CAA ACT GAT GAC ACA   270
                             FR3

A   I   Y   Y   C   A   S   G   P   Y   Y   Y   T   M   D   Y   W   G     108
GCC ATA TAT TAC TGT GCC AGT GGT CCT TAT TAC TAT ACT ATG GAC TAC TGG GGT   324
      FR3                               CDR3

Q   G   T   S   V   T   V   S   S                                         117
CAA GGA ACC TCA GTC ACC GTC TCC TCA                                       351
         FR4
```

**Fig. 2**

```
GCT AGC ACC AAG GGC CCA TCG GTC TTC CCC CTG GCA CCC TCC TCC AAG    48
 A   S   T   K   G   P   S   V   F   P   L   A   P   S   S   K     16

AGC ACC TCT GGG GGC ACA GCG GCC CTG GGC TGC CTG GTC AAG GAC TAC    96
 S   T   S   G   G   T   A   A   L   G   C   L   V   K   D   Y     32

TTC CCC GAA CCG GTG ACG GTG TCG TGG AAC TCA GGC GCC CTG ACC AGC   144
 F   P   E   P   V   T   V   S   W   N   S   G   A   L   T   S     48

GGC GTG CAC ACC TTC CCG GCT GTC CTA CAG TCC TCA GGA CTC TAC TCC   192
 G   V   H   T   F   P   A   V   L   Q   S   S   G   L   Y   S     64

CTC AGC AGC GTG GTG ACC GTG CCC TCC AGC AGC TTG GGC ACC CAG ACC   240
 L   S   S   V   V   T   V   P   S   S   S   L   G   T   Q   T     80

TAC ATC TGC AAC GTG AAT CAC AAG CCC AGC AAC ACC AAG GTG GAC AAG   288
 Y   I   C   N   V   N   H   K   P   S   N   T   K   V   D   K     96

AAA GTT GAG CCC AAA TCT TGT GAC AAA ACT CAC ACA TGC CCA CCG TGC   336
 K   V   E   P   K   S   C   D   K   T   H   T   C   P   P   C    112

CCA TGC TAA                                                       345
 P   C   *                                                        115
```

**Fig. 3**

```
GCT AGC ACC AAG GGC CCA TCG GTC TTC CCC CTG GCA CCC TCC TCC AAG    48
 A   S   T   K   G   P   S   V   F   P   L   A   P   S   S   K     16

AGC ACC TCT GGG GGC ACA GCG GCC CTG GGC TGC CTG GTC AAG GAC TAC    96
 S   T   S   G   G   T   A   A   L   G   C   L   V   K   D   Y     32

TTC CCC GAA CCG GTG ACG GTG TCG TGG AAC TCA GGC GCC CTG ACC AGC   144
 F   P   E   P   V   T   V   S   W   N   S   G   A   L   T   S     48

GGC GTG CAC ACC TTC CCG GCT GTC CTA CAG TCC TCA GGA CTC TAC TCC   192
 G   V   H   T   F   P   A   V   L   Q   S   S   G   L   Y   S     64

CTC AGC AGC GTG GTG ACC GTG CCC TCC AGC AGC TTG GGC ACC CAG ACC   240
 L   S   S   V   V   T   V   P   S   S   S   L   G   T   Q   T     80

TAC ATC TGC AAC GTG AAT CAC AAG CCC AGC AAC ACC AAG GTG GAC AAG   288
 Y   I   C   N   V   N   H   K   P   S   N   T   K   V   D   K     96

AAA GTT GAG CCC AAA TCT TGT GAC AAA ACT CAC ACA TGC CCA CCG TGC   336
 K   V   E   P   K   S   C   D   K   T   H   T   C   P   P   C    112

CCA GCA CCT GAA CTC CTG GGG GGA CCG TCA GTC TTC CTC TTC CCC CCA   384
 P   A   P   E   L   L   G   G   P   S   V   F   L   F   P   P    128

AAA CCC AAG GAC ACC CTC ATG ATC TCC CGG ACC CCT GAG GTC ACA TGC   432
 K   P   K   D   T   L   M   I   S   R   T   P   E   V   T   C    144

GTG GTG GTG GAC GTG AGC CAC GAA GAC CCT GAG GTC AAG TTC AAC TGG   480
 V   V   V   D   V   S   H   E   D   P   E   V   K   F   N   W    160

TAC GTG GAC GGC GTG GAG GTG CAT AAT GCC AAG ACA AAG CCG CGG GAG   528
 Y   V   D   G   V   E   V   H   N   A   K   T   K   P   R   E    176

GAG CAG TAC AAC AGC ACG TAC CGT GTG GTC AGC GTC CTC ACC GTC CTG   576
 E   Q   Y   N   S   T   Y   R   V   V   S   V   L   T   V   L    192

CAC CAG GAC TGG CTG AAT GGC AAG GAG TAC AAG TGC AAG GTC TCC AAC   624
 H   Q   D   W   L   N   G   K   E   Y   K   C   K   V   S   N    208

AAA GCC CTC CCA GCC CCC ATC GAG AAA ACC ATC TCC AAA GCC AAA GGG   672
 K   A   L   P   A   P   I   E   K   T   I   S   K   A   K   G    224

CAG CCC CGA GAA CCA CAG GTG TAC ACC CTG CCC CCA TCC CGG GAT GAG   720
 Q   P   R   E   P   Q   V   Y   T   L   P   P   S   R   D   E    240

CTG ACC AAG AAC CAG GTC AGC CTG ACC TGC CTG GTC AAA GGC TTC TAT   768
 L   T   K   N   Q   V   S   L   T   C   L   V   K   G   F   Y    256

CCC AGC GAC ATC GCC GTG GAG TGG GAG AGC AAT GGG CAG CCG GAG AAC   816
 P   S   D   I   A   V   E   W   E   S   N   G   Q   P   E   N    272

AAC TAC AAG ACC ACG CCT CCC GTG CTG GAC TCC GAC GGC TCC TTC TTC   864
 N   Y   K   T   T   P   P   V   L   D   S   D   G   S   F   F    288

CTC TAC AGC AAG CTC ACC GTG GAC AAG AGC AGG TGG CAG CAG GGG AAC   912
 L   Y   S   K   L   T   V   D   K   S   R   W   Q   Q   G   N    304

GTC TTC TCA TGC TCC GTG ATG CAT GAG GCT CTG CAC AAC CAC TAC ACG   960
 V   F   S   C   S   V   M   H   E   A   L   H   N   H   Y   T    320

CAG AAG AGC CTC TCC CTG TCT CCG GGT AAA TAA                        990
 Q   K   S   L   S   L   S   P   G   K   *                         330
```

**Fig. 4**

```
GCT AGC ACC AAG GGC CCA TCC GTC TTC CCC CTG GCG CCC TGC TCC AGG    48
 A   S   T   K   G   P   S   V   F   P   L   A   P   C   S   R     16

AGC ACC TCC GAG AGC ACA GCC GCC CTG GGC TGC CTG GTC AAG GAC TAC    96
 S   T   S   E   S   T   A   A   L   G   C   L   V   K   D   Y     32

TTC CCC GAA CCG GTG ACG GTG TCG TGG AAC TCA GGC GCC CTG ACC AGC   144
 F   P   E   P   V   T   V   S   W   N   S   G   A   L   T   S     48

GGC GTG CAC ACC TTC CCG GCT GTC CTA CAG TCC TCA GGA CTC TAC TCC   192
 G   V   H   T   F   P   A   V   L   Q   S   S   G   L   Y   S     64

CTC AGC AGC GTG GTG ACC GTG CCC TCC AGC AGC TTG GGC ACG AAG ACC   240
 L   S   S   V   V   T   V   P   S   S   S   L   G   T   K   T     80

TAC ACC TGC AAC GTA GAT CAC AAG CCC AGC AAC ACC AAG GTG GAC AAG   288
 Y   T   C   N   V   D   H   K   P   S   N   T   K   V   D   K     96

AGA GTT GAG TCC AAA TAT GGT CCC CCA TGC CCA TCA TGC CCA GCA CCT   336
 R   V   E   S   K   Y   G   P   P   C   P   S   C   P   A   P    112

GAG TTC CTG GGG GGA CCA TCA GTC TTC CTG TTC CCC CCA AAA CCC AAG   384
 E   F   L   G   G   P   S   V   F   L   F   P   P   K   P   K    128

GAC ACT CTC ATG ATC TCC CGG ACC CCT GAG GTC ACG TGC GTG GTG GTG   432
 D   T   L   M   I   S   R   T   P   E   V   T   C   V   V   V    144

GAC GTG AGC CAG GAA GAC CCC GAG GTC CAG TTC AAC TGG TAC GTG GAT   480
 D   V   S   Q   E   D   P   E   V   Q   F   N   W   Y   V   D    160

GGC GTG GAG GTG CAT AAT GCC AAG ACA AAG CCG CGG GAG GAG CAG TTC   528
 G   V   E   V   H   N   A   K   T   K   P   R   E   E   Q   F    176

AAC AGC ACG TAC CGT GTG GTC AGC GTC CTC ACC GTC CTG CAC CAG GAC   576
 N   S   T   Y   R   V   V   S   V   L   T   V   L   H   Q   D    192

TGG CTG AAC GGC AAG GAG TAC AAG TGC AAG GTC TCC AAC AAA GGC CTC   624
 W   L   N   G   K   E   Y   K   C   K   V   S   N   K   G   L    208

CCG TCC TCC ATC GAG AAA ACC ATC TCC AAA GCC AAA GGG CAG CCC CGA   672
 P   S   S   I   E   K   T   I   S   K   A   K   G   Q   P   R    224

GAG CCA CAG GTG TAC ACC CTG CCC CCA TCC CAG GAG GAG ATG ACC AAG   720
 E   P   Q   V   Y   T   L   P   P   S   Q   E   E   M   T   K    240

AAC CAG GTC AGC CTG ACC TGC CTG GTC AAA GGC TTC TAC CCC AGC GAC   768
 N   Q   V   S   L   T   C   L   V   K   G   F   Y   P   S   D    256

ATC GCC GTG GAG TGG GAG AGC AAT GGG CAG CCG GAG AAC AAC TAC AAG   816
 I   A   V   E   W   E   S   N   G   Q   P   E   N   N   Y   K    272

ACC ACG CCT CCC GTG CTG GAC TCC GAC GGC TCC TTC TTC CTC TAC AGC   864
 T   T   P   P   V   L   D   S   D   G   S   F   F   L   Y   S    288

AGG CTA ACC GTG GAC AAG AGC AGG TGG CAG GAG GGG AAT GTC TTC TCA   912
 R   L   T   V   D   K   S   R   W   Q   E   G   N   V   F   S    304

TGC TCC GTG ATG CAT GAG GCT CTG CAC AAC CAC TAC ACA CAG AAG AGC   960
 C   S   V   M   H   E   A   L   H   N   H   Y   T   Q   K   S    320

CTC TCC CTG TCT CTG GGT AAA TAA                                  981
 L   S   L   S   L   G   K   *                                   327
```

**Fig. 5**

```
ACG GTG GCT GCA CCA TCT GTC TTC ATC TTC CCG CCA TCT GAT GAG CAG    48
 T   V   A   A   P   S   V   F   I   F   P   P   S   D   E   Q     16

TTG AAA TCT GGA ACT GCC TCT GTT GTG TGC CTG CTG AAT AAC TTC TAT    96
 L   K   S   G   T   A   S   V   V   C   L   L   N   N   F   Y     32

CCC AGA GAG GCC AAA GTA CAG TGG AAG GTG GAT AAC GCC CTC CAA TCG   144
 P   R   E   A   K   V   Q   W   K   V   D   N   A   L   Q   S     48

GGT AAC TCC CAG GAG AGT GTC ACA GAG CAG GAC AGC AAG GAC AGC ACC   192
 G   N   S   Q   E   S   V   T   E   Q   D   S   K   D   S   T     64

TAC AGC CTC AGC AGC ACC CTG ACG CTG AGC AAA GCA GAC TAC GAG AAA   240
 Y   S   L   S   S   T   L   T   L   S   K   A   D   Y   E   K     80

CAC AAA GTC TAC GCC TGC GAA GTC ACC CAT CAG GGC CTG AGT TCG CCC   288
 H   K   V   Y   A   C   E   V   T   H   Q   G   L   S   S   P     96

GTC ACA AAG AGC TTC AAC AGG GGA GAG TGT TAA                       321
 V   T   K   S   F   N   R   G   E   C   *                        107
```

**Fig. 6**

```
ATG GAC ATG CGT GTG CCC GCT CAA CTC CTG GGC CTG CTG CTG CTC TGG    48
 M   D   M   R   V   P   A   Q   L   L   G   L   L   L   L   W     16

CTC CCA GGT GCG CGC TGT                                            66
 L   P   G   A   R   C                                             22
```

**Fig. 7**

```
ATG GAG TTC GGC CTG AGC TGG CTG TTC CTG GTG GCT ATT CTT AAG GGT    48
 M   E   F   G   L   S   W   L   F   L   V   A   I   L   K   G     16

GTC CAG TGT                                                        57
 V   Q   C                                                         19
```

**Fig. 8**

GCCAAAACAACAGCCCCATCGGTCTATCCACTGGCCCCTGTGTGTGGAGATACAACTGGCTCCTCGGTGA
CTCTAGGATGCCTGGTCAAGGGTTATTTCCCTGAGCCAGTGACCTTGACCTGGAACTCTGGATCCCTGTC
CAGTGGTGTGCACACCTTCCCAGCTGTCCTGCAGTCTGACCTCTACACCCTCAGCAGCTCAGTGACTGTA
ACCTCGAGCACCTGGCCCAGCCAGTCCATCACCTGCAATGTGGCCCACCCGGCAAGCAGCACCAAGGTGG
ACAAGAAAATTGAGCCCAGAGGGCCCACAATCAAGCCCTGTCCTCCATGCAAATGCCCAGCACCTAACCT
CTTGGGTGGACCATCCGTCTTCATCTTCCCTCCAAAGATCAAGGATGTACTCATGATCTCCCTGAGCCCC
ATAGTCACATGTGTGGTGGTGGATGTGAGCGAGGATGACCCAGATGTCCAGATCAGCTGGTTTGTGAACA
ACGTGGAAGTACACACAGCTCAGACACAAACCCATAGAGAGGATTACAACAGTACTCTCCGGGTGGTCAG
TGCCCTCCCCATCCAGCACCAGGACTGGATGAGTGGCAAGGAGTTCAAATGCAAGGTCAACAACAAAGAC
CTCCCAGCGCCCATCGAGAGAACCATCTCAAAACCCAAAGGGTCAGTAAGAGCTCCACAGGTATATGTCT
TGCCTCCACCAGAAGAAGAGATGACTAAGAAACAGGTCACTCTGACCTGCATGGTCACAGACTTCATGCC
TGAAGACATTTACGTGGAGTGGACCAACAACGGGAAAACAGAGCTAAACTACAAGAACACTGAACCAGTC
CTGGACTCTGATGGTTCTTACTTCATGTACAGCAAGCTGAGAGTGGAAAAGAAGAACTGGGTGGAAAGAA
ATAGCTACTCCTGTTCAGTGGTCCACGAGGGTCTGCACAATCACCACACGACTAAGAGCTTCTCCCGGAC
TCCGGGTAAATGAGCTCAGCACCCACAAAACTCTCAGGTCCAAAGAGACACCCACACTCATCTCCATGCT
TCCCTTGTATAAATAAAGCACCCAGCAATGCCTGGGACCATGTAA

**Fig. 9A**

AKTTAPSVYPLAPVCGDTTGSSVTLGCLVKGYFPEPVTLTWNSGSLSSGVHTFPAVLQSD
LYTLSSSVTVTSSTWPSQSITCNVAHPASSTKVDKKIEPRGPTIKPCPPCKCPAPNLLGG
PSVFIFPPKIKDVLMISLSPIVTCVVVDVSEDDPDVQISWFVNNVEVHTAQTQTHREDYN
STLRVVSALPIQHQDWMSGKEFKCKVNNKDLPAPIERTISKPKGSVRAPQVYVLPPPEEE
MTKKQVTLTCMVTDFMPEDIYVEWTNNGKTELNYKNTEPVLDSDGSYFMYSKLRVEKKNW
VERNSYSCSVVHEGLHNHHTTKSFSRTPGK

**Fig. 9B**

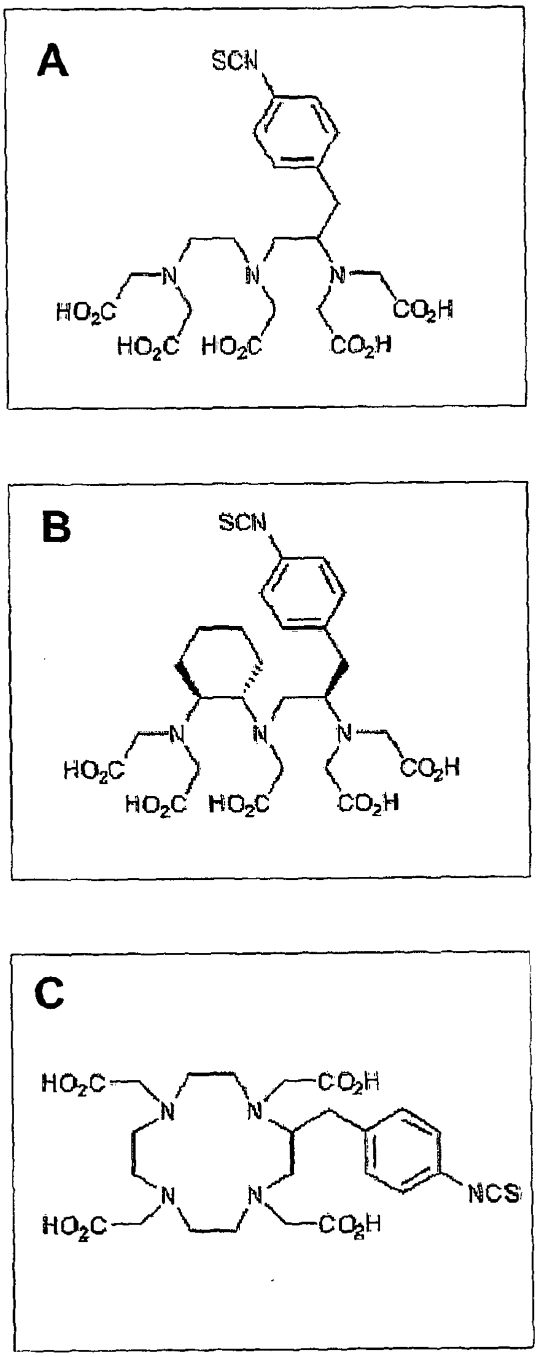

Fig. 10

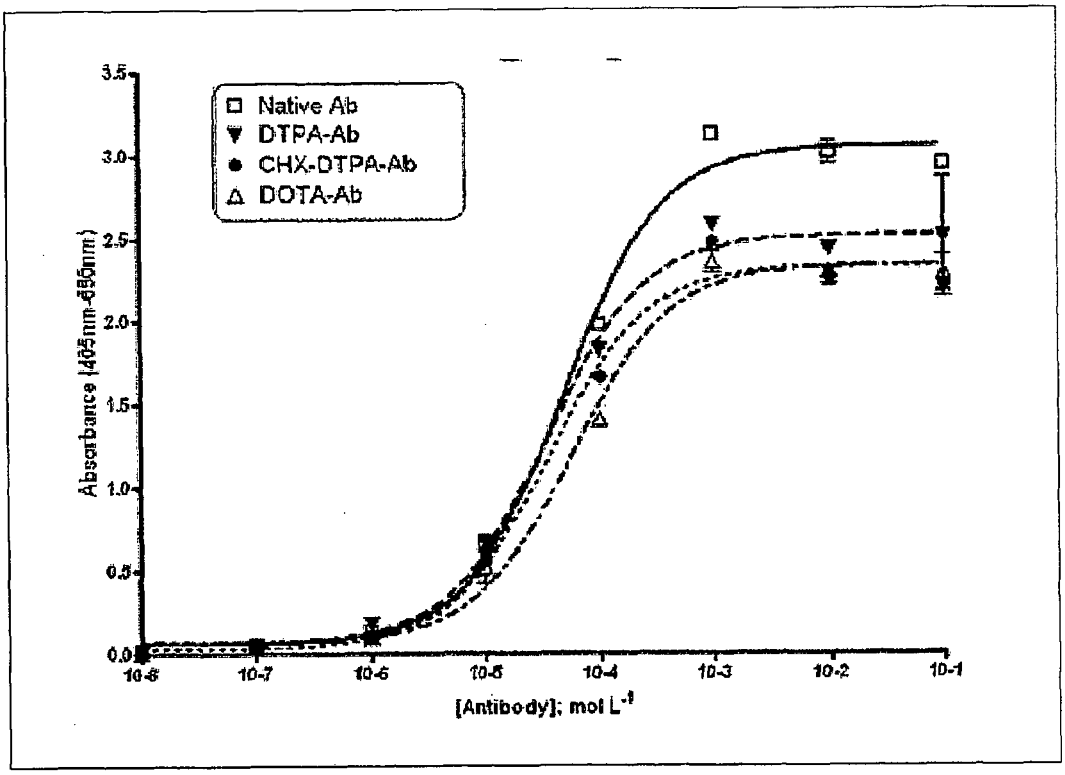
Native Ab
DTPA-Ab
CHX-DTPA-Ab
DOTA-Ab
Absorbance (405nm-650nm)
3.5
3.0
2.5
2.0
1.5
1.0
0.5
0.0
10-8
10-7
10-6
10-5
10-4
10-3
10-2
10-1
[Antibody]; mol L-1

Fig. 11

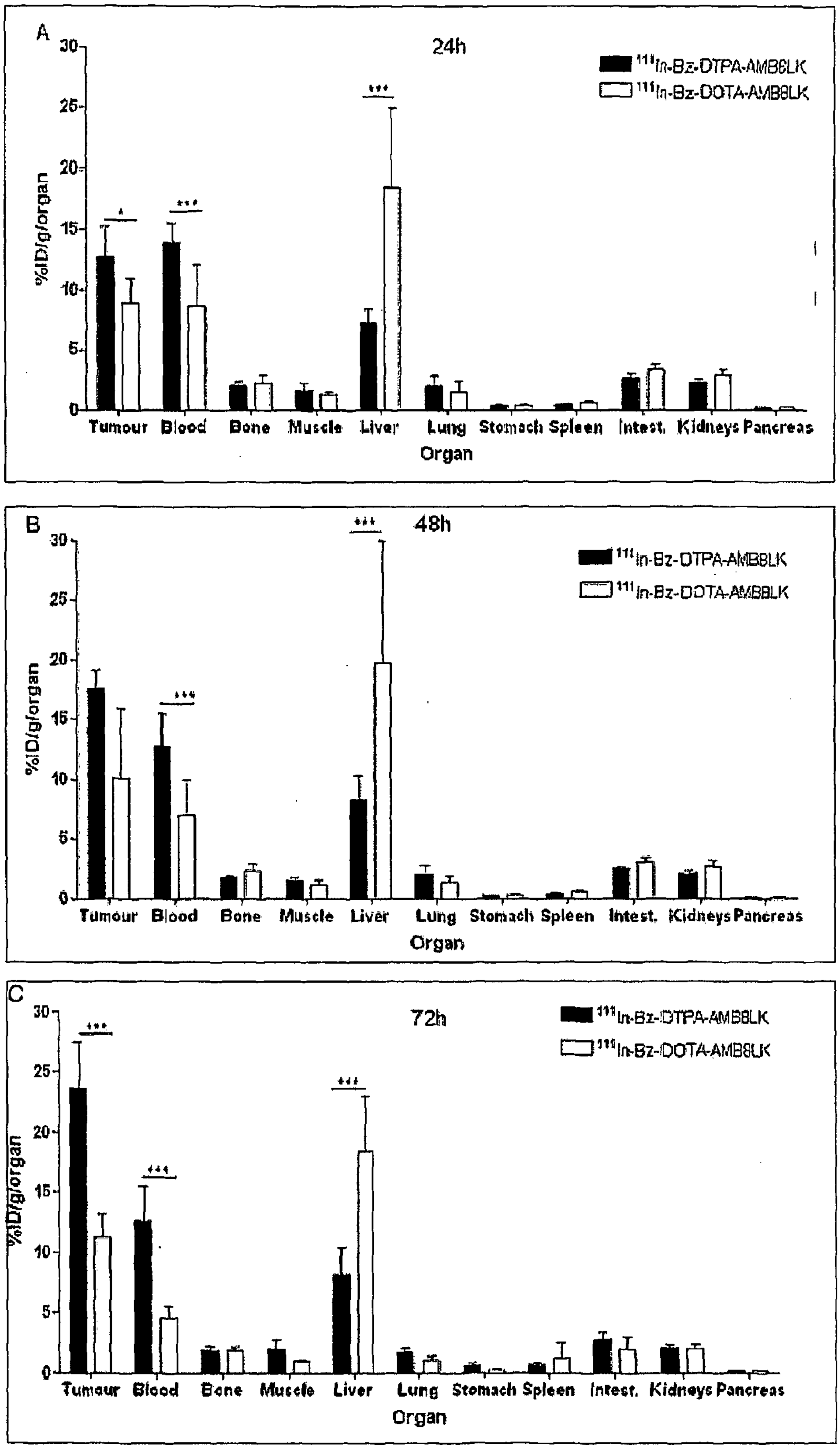
A
24h
$^{111}$In-Bz-DTPA-AMB8LK
$^{111}$In-Bz-DOTA-AMB8LK
%ID/g/organ
Tumour Blood Bone Muscle Liver Lung Stomach Spleen Intest. Kidneys Pancreas
Organ
B
48h
$^{111}$In-Bz-DTPA-AMB8LK
$^{111}$In-Bz-DOTA-AMB8LK
%ID/g/organ
Tumour Blood Bone Muscle Liver Lung Stomach Spleen Intest. Kidneys Pancreas
Organ
C
72h
$^{111}$In-Bz-DTPA-AMB8LK
$^{111}$In-Bz-DOTA-AMB8LK
%ID/g/organ
Tumour Blood Bone Muscle Liver Lung Stomach Spleen Intest. Kidneys Pancreas
Organ

Fig. 12

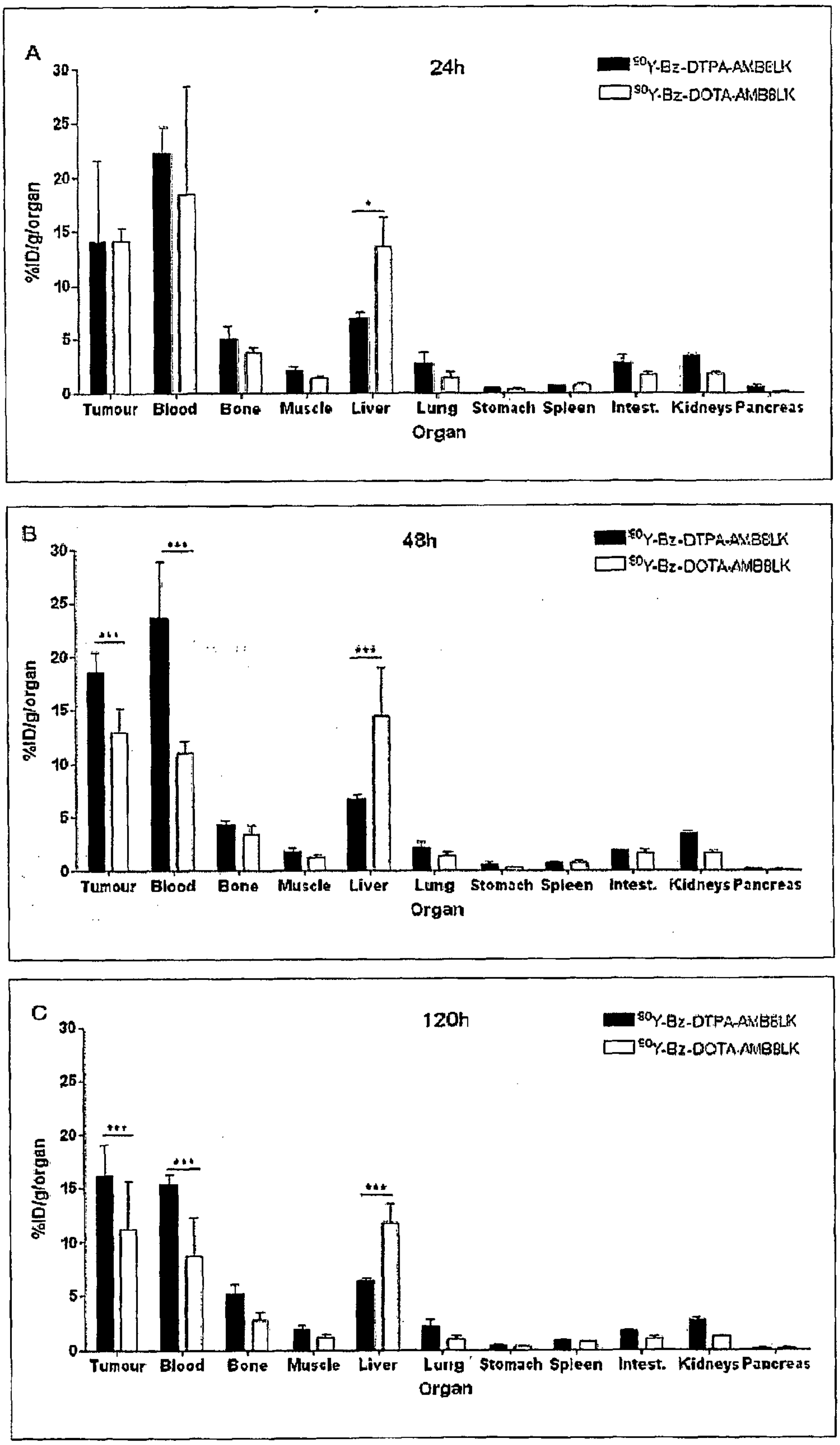
A
24h
B
48h
C
120h
$^{90}$Y-Bz-DTPA-AMB8LK
$^{90}$Y-Bz-DOTA-AMB8LK
%ID/g/organ
Tumour Blood Bone Muscle Liver Lung Stomach Spleen Intest. Kidneys Pancreas
Organ
0
5
10
15
20
25
30

Fig. 13

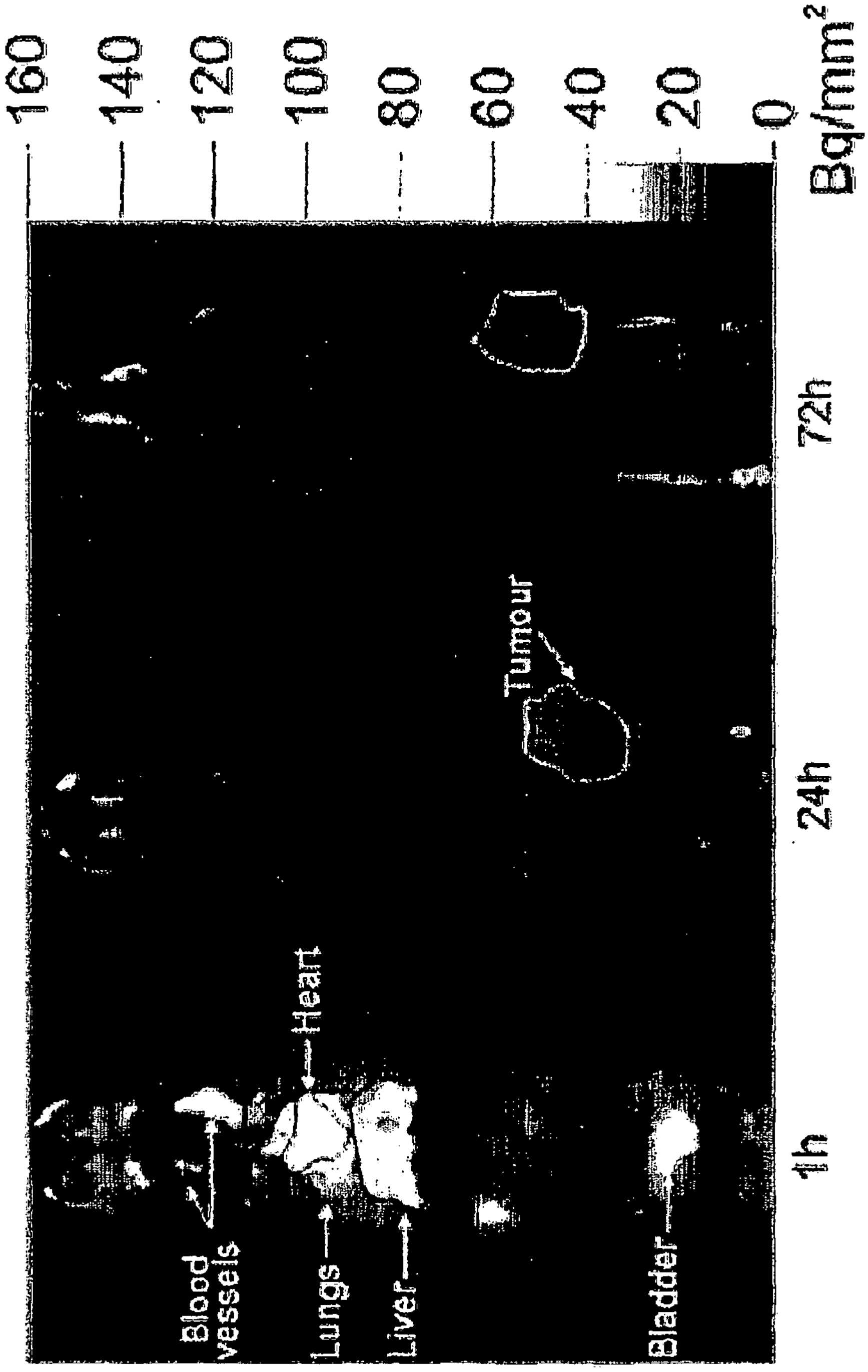
160
140
120
100
80
60
40
20
0
Bq/mm2
Tumour
1h
24h
72h
Blood vessels
Heart
Lungs
Liver
Bladder

Fig. 14

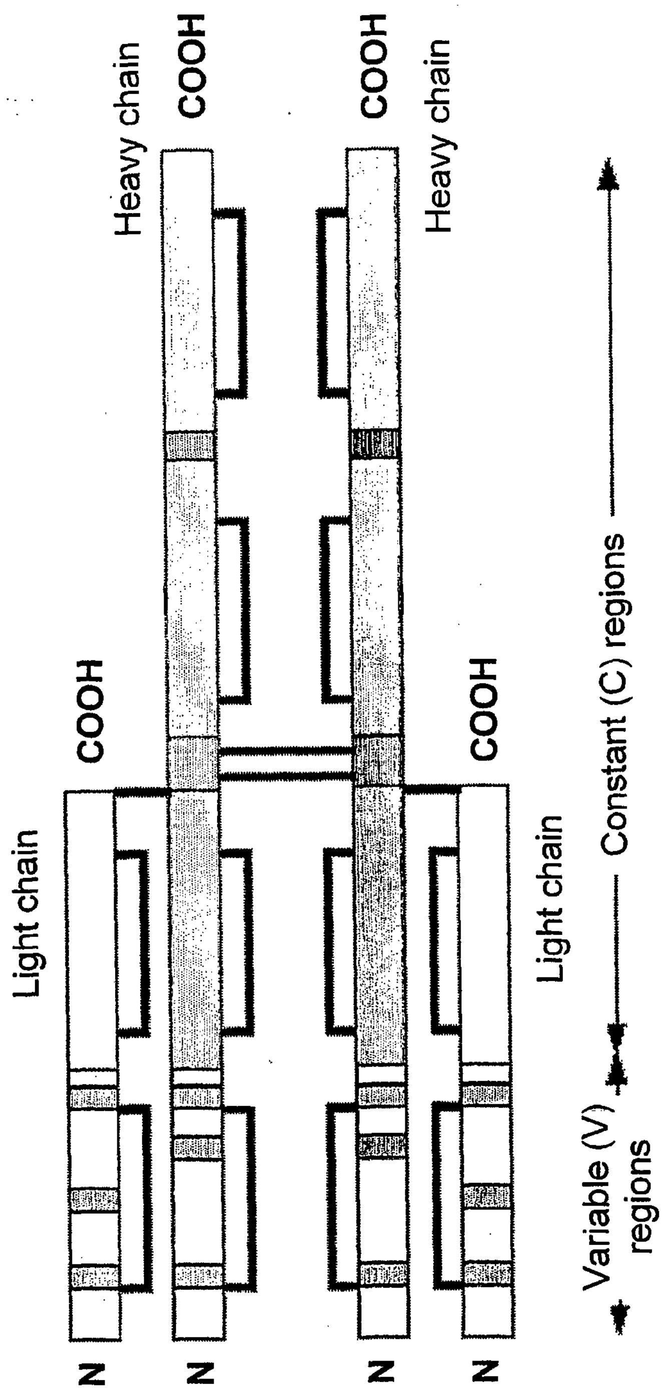
Heavy chain
COOH
COOH
Heavy chain
Light chain
COOH
COOH
Light chain
N
N
N
N
Variable (V) regions
Constant (C) regions

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8804936 A
- US 7153506 B
- WO 0152889 A
- US 7098308 B
- US 5255212 A


### Non-patent literature cited in the description


- **GUNER et al.** Cytosol and serum ferritin in breast carcinoma. *Cancer Lett,* 1992, vol. 67 (2-3), 103-112
- **WEINSTEIN et al.** Tissue ferritin concentration and prognosis in carcinoma of the breast. *Breast Cancer Res Treat,* 1989, vol. 14 (3), 349-53
- **ESHHAR et al.** Ferritin, a Hodkin;s disease Associated Antigen. *PNAS U.S.A.,* 1974, vol. 71 (10), 3956-60
- **DRYSDALE et al.** Human isoferritins in normal and disease states. *Semin Hematol,* 1977, vol. 14 (1), 71-88
- **MARCUS et al.** Isolation of ferritin from human mammary and pancreatic carcinomas by means of antibody immunoadsorbents. *Arch Biochem Biophys,* 1974, vol. 162 (2), 493-501
- **VRIESENDORP et al.** Radiolabelled immunoglobulin therapy in patients with Hodgkin's disease. *Cancer Biother Radiopharm,* 2000, vol. 15 (5), 431-45
- **VRIESENDORP et al.** Review of five consecutive studies of radiolabeled immunoglobulin therapy in Hodgkin's disease. *Cancer Res,* 1995, vol. 55 (23), 5888s92s
- **SABBAH et al.** In vitro and in vivo comparison of DTPA- and DOTA-conjugated antiferritin monoclonal antibody for imaging and therapy of pancreatic cancer. *Nuclear Medicine and Biology,* vol. 34 (3), 293-304
- **KADOUCHE et al.** Analysis of various isoferritins with monoclonal antibodies. *C R Seances Acad Sci III,* 1982, vol. 295 (6), 443-448
- **JEFFREY et al.** Development and Properties of β-Glucuronide linkers for Monoclonal Antibody-Drug conjugates. *Bioconjugate Chem.,* 2006, vol. 17 (3), 831-840
- Remington's Pharmaceutical Sciences. 1980
- Controlled release of bioactive agents from lactide/glycolide polymer. **LEWIS.** Biodegradble Polymers as Drug Delivery Sysytems. Marcel Decker, 1990
- **HUNKAPILLER et al.** *Nature,* vol. 310, 105-111
- **GOEDDEL, D. et al.** *Nucleic Acid Res.,* 1980, vol. 8, 4057
- **KADOUCHE et al.** Analysis of various isoferritins with monoclonal antibodies. *C R Seances Acad Sci III,* 1982, vol. 295 (6), 443-8
- **COOPER et al.** Conjugation of chelating agents to proteins and radiolabeling with trivalent metallic isotopes. *Nature Protocols,* 2006, vol. 1, 314-17
- **MEARES et al.** Conjugation of antibodies with bifunctional chelating agents: isothiocyanate andbromoacetamide reagents, methods of analysis, and subsequent addition of metal ions. *Anal Biochem,* 1984, vol. 142 (1), 68-78
- **LINDMO et al.** Determination of the immunoreactive fraction of radiolabeled monoclonal antibodies by linear extrapolation to binding at infinite antigen excess. *J Immunol Methods,* 1984, vol. 72 (1), 77-89
- **KYRIAZIS et al.** Human pancreatic adenocarcinoma line Capan-1 in tissue culture and the nude mouse:morphologic, biologic, and biochemical characteristics. *Am J Pathol,* 1982, vol. 106 (2), 250-60
- **GOLDSTEIN et al.** The design and evaluation of a novel targeted drug delivery system using cationic emulsion-antibody conjugates. *J Control Release,* 2005, vol. 108 (2-3), 418-32